(19) Europäisches Patentamt
European Patent Office
Office européen des brevets

(11) Veröffentlichungsnummer: **0 486 804 A2**

# (12) EUROPÄISCHE PATENTANMELDUNG

(21) Anmeldenummer: **91117254.2**

(22) Anmeldetag: **10.10.91**

(51) Int. Cl.5: **C07D 413/12**, C07D 403/12, C07D 417/12, C07D 401/12, A01N 43/80, A01N 43/50, A01N 43/82, //(C07D413/12, 261:00,231:00),(C07D403/12, 233:00,231:00),(C07D413/12, 271:00,231:00),(C07D417/12, 285:00,231:00),(C07D413/12, 261:00,213:00),(C07D401/12, 233:00,213:00)

(30) Priorität: **20.10.90 DE 4033484**

(43) Veröffentlichungstag der Anmeldung:
**27.05.92 Patentblatt 92/22**

(84) Benannte Vertragsstaaten:
**AT BE CH DE DK ES FR GB IT LI NL**

(71) Anmelder: **BASF Aktiengesellschaft**
**Carl-Bosch-Strasse 38**
**W-6700 Ludwigshafen(DE)**

(72) Erfinder: **Nuebling, Christoph, Dr.**
**Wilhelmstrasse 13**
**W-6733 Hassloch(DE)**
Erfinder: **Theobald, Hans, Dr.**
**Oueichstrasse 6**
**W-6703 Limburgerhof(DE)**
Erfinder: **Krieg, Wolfgang, Dr.**
**Saarstrasse 17**
**W-6721 Weingarten(DE)**
Erfinder: **Kardorff, Uwe, Dr.**
**D 3,4**
**W-6800 Mannheim 1(DE)**
Erfinder: **Kuenast, Christoph, Dr.**
**Salierstrasse 2**
**W-6701 Otterstadt(DE)**

## (54) Hydrochinondiether, Verfahren zu ihrer Herstellung und ihre Verwendung zur Schädlingsbekämpfung.

(57) Hydrochinondiether der allgemeinen Formel I,

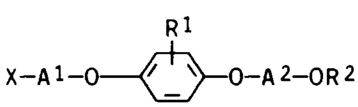

I

in der die Substituenten folgende Bedeutung haben:
$A^1$, $A^2$
ggf. subst. Alkylen;
$R^1$
Wasserstoff, Halogen oder Alkyl;
$R^2$
ggf. subst. Pyrazol oder Pyridyl;
X



Rank Xerox (UK) Business Services

ggf. subst. fünfgliedriger Heteroaromat,
Verfahren zu ihrer Herstellung und dafür verwendete Zwischenprodukte sowie die Verwendung der Verbindungen I.

Die vorliegende Erfindung betrifft Hydrochinondiether der allgemeinen Formel I,

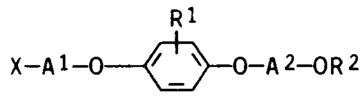

I

in der die Substituenten folgende Bedeutung haben:

$A^1$

Methylen, Ethylen oder Propylen, wobei diese Gruppen ein oder zwei $C_1$-$C_3$-Alkylreste tragen können;

$A^2$

Ethylen oder Propylen, wobei diese Gruppen ein oder zwei $C_1$-$C_3$-Alkylreste tragen können;

$R^1$

Wasserstoff, Halogen oder $C_1$-$C_6$-Alkyl;

$R^2$

1-Pyrazolyl, welches ein bis drei der folgenden Reste tragen kann: Halogen und $C_1$-$C_3$-Alkyl,

2-, 3- oder 4-Pyridyl, welches ein bis drei der folgenden Reste tragen kann: Cyano, Nitro, Halogen, $C_1$-$C_3$-Alkyl, $C_1$-$C_2$-Halogenalkyl, $C_1$-$C_3$-Alkoxy, $C_1$-$C_2$-Halogenalkoxy, $C_1$-$C_3$-Alkylthio, $C_1$-$C_3$-Alkoxy-$C_1$-$C_3$-alkyl oder $C_3$-$C_6$-Cycloalkyl;

X

ein fünfgliedriger Heteroaromat, enthaltend ein bis drei Stickstoffatome und/oder ein Sauerstoff- oder Schwefelatom als Heteroatome, welcher ein bis drei der folgenden Reste tragen kann: Nitro, Halogen, $C_1$-$C_6$-Alkyl, $C_1$-$C_4$-Halogenalkyl, $C_1$-$C_6$-Alkoxy, $C_1$-$C_4$-Halogenalkoxy, $C_1$-$C_4$-Alkylthio, $C_1$-$C_4$-Halogenalkylthio, $C_1$-$C_3$-Alkoxy-$C_1$-$C_4$-alkyl, $C_3$-$C_8$-Cycloalkyl, $C_2$-$C_8$-Alkenyl, Aryl oder Aryl-$C_1$-$C_{10}$-Alkyl, wobei die aromatischen Reste ihrerseits ein bis fünf Halogenatome und/oder ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio.

Außerdem betrifft die Erfindung Verfahren zur Herstellung dieser Verbindungen, sie enthaltende Schädlingsbekämpfungsmittel sowie Verfahren zur Schädlingsbekämpfung.

Desweiteren betrifft die Erfindung Zwischenprodukte zur Herstellung der Hydrochinondiether I, nämlich Diether der allgemeinen Formel IV

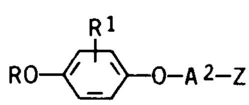

IV

in der $A^2$ und $R^1$ die vorstehende Bedeutung haben und die weiteren Substituenten für folgende Reste stehen:

R

$C_1$-$C_6$-Alkyl;

Benzyl, wobei der aromatische Rest ein bis fünf Halogenatome und/oder ein bis drei der folgenden Gruppen tragen kann: Cyano, Nitro, $C_1$-$C_4$-Alkyl, $C_1$-$C_4$-Alkoxy oder $C_1$-$C_4$-Alkylthio;

Silyl, welches drei der folgenden Reste trägt: $C_1$-$C_6$-Alkyl und/oder Phenyl, wobei der aromatische Rest ein bis fünf Halogenatome und/oder ein bis drei der folgenden Gruppen tragen kann: Cyano, Nitro, $C_1$-$C_4$-Alkyl, $C_1$-$C_4$-Alkoxy oder $C_1$-$C_4$-Alkylthio;

Z

Hydroxy; Halogen;

$C_1$-$C_{10}$-Alkylsulfonyl, Phenylsulfonyl, wobei der aromatische Rest ein bis fünf Halogenatome und/oder ein bis drei der folgenden Gruppen tragen kann: Cyano, Nitro, $C_1$-$C_4$-Alkyl, $C_1$-$C_4$-Alkoxy oder $C_1$-$C_4$-Alkylthio;

eine Pyrazolyl-1-oxy-gruppe, welche ein bis drei der folgenden Reste tragen kann: Halogen und $C_1$-$C_3$-Alkyl,

oder eine Pyridyl-2-, -3- oder -4-oxy-gruppe, welche ein bis drei der folgenden Reste tragen kann: Cyano, Nitro, Halogen, $C_1$-$C_3$-Alkyl, $C_1$-$C_2$-Halogenalkyl, $C_1$-$C_3$-Alkoxy, $C_1$-$C_2$-Halogenalkoxy, $C_1$-$C_3$-Alkylthio, $C_1$-$C_3$-Alkoxy-$C_1$-$C_3$-alkyl oder $C_3$-$C_6$-Cycloalkyl,

und Monoether der allgemeinen Formel VI

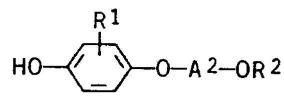

VI

in der $A^2$, $R^1$ und $R^2$ die vorstehend gegebene Bedeutung haben.

Aus der Literatur sind Hydrochinondiether mit schädlingsbekämpfender Wirkung bekannt (Phenoxyphenoxyalkyl-O-oximether: GB-A 21 15 812; Phenoxyphenoxyalkyl-pyrazole: EP-A 289 919; Phenoxyphenoxyalkyl-hetarylether: GB-A 21 40 010; Phenoxyphenoxyalkyl-N-pyrazolyl- bzw. -N-Triazolylether: DE-A 39 06 772 und DE-A 39 41 296), deren Wirkung gegen Schädlinge beispielsweise bei niedrigen Aufwandmengen nicht immer befriedigt.

Aufgabe der vorliegenden Erfindung waren neue, zur Schädlingsbekämpfung geeignete Verbindungen und Verfahren zu ihrer Herstellung und ihrer Verwendung.

Demgemäß wurden die eingangs definierten Hydrochinondiether I gefunden. Außerdem wurden Verfahren und neue Zwischenprodukte zur Herstellung dieser Hydrochinondiether, sie enthaltende Schädlingsbekämpfungsmittel und Verfahren zu ihrer Verwendung gefunden.

Die Hydrochinondiether I sind auf verschiedenen Wegen zugänglich.

Besonders vorteilhaft erhält man sie in an sich bekannter Weise, indem man einen 4-Hydroxyphenolether II in einem inerten organischen Lösungsmittel in Gegenwart einer Base mit einem Ethylen- oder Propylenderivat III zu einem Diether IVa verethert, IVa anschließend mit einer Hydroxyverbindung V verethert, das so erhaltene Dietherderivat IVb durch Abspaltung der Schutzgruppe R in den entsprechenden Monoether VI überführt, welcher anschließend mit einer Arylalkylverbindung VII zu I verethert wird.

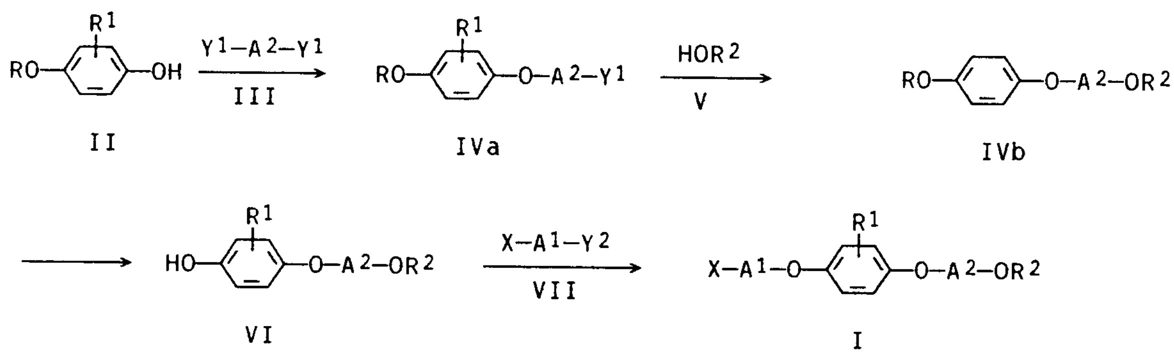


II IVa IVb VI I

R in den Formeln II, IVa und IVb steht für eine inerte Schutzgruppe wie $C_1$-$C_6$-Alkyl, insbesondere 1,1-Dimethylethyl, im Phenylteil unsubstituiertes oder substituiertes Benzyl, insbesondere Benzyl, 4-Methylbenzyl und 2,4,6-Trimethylbenzyl, dreifach durch C-organische Reste substituiertes Silyl, vorzugsweise Trimethylsilyl, tert.-Butyl-dimethylsilyl, Phenyldimethylsilyl und tert.-Butyl-dipenylsilyl, insbesondere tert.-Butyldimethylsilyl, $C_1$-$C_4$-Alkoxy-$C_1$-$C_2$-alkyl wie Methoxymethyl, Ethoxymethyl, 1-Methoxyethyl und 1-Ethoxyethyl, insbesondere Methoxymethyl oder Methoxyethoxymethyl.

$Y^1$ bzw. $Y^2$ in den Formeln III, IVa bzw. VII stehen jeweils für nucleofuge Abgangsgruppen, beispielsweise Halogen wie Fluor, Chlor, Brom und Iod, vorzugsweise Chlor, Brom und Iod, insbesondere Chlor und Brom, durch C-organische Reste substituiertes Sulfonyl, insbesondere Methylsulfonyl, Trifluormethylsulfonyl, Phenylsulfonyl, 4-Tolylsulfonyl und 2,4,6-Trimethylphenylsulfonyl oder $C_1$-$C_4$-Alkylcarbonyloxy wie Methylcarbonyloxy (Acetyl), Ethylcarbonyloxy (Propionyl), 1-Methylethylcarbonyloxy, welches ein bis fünf Fluoratome tragen kann, beispielsweise Trifluormethylcarbonyloxy (Trifluoracetyl), Benzoyl, welches ein bis drei Halogenatome wie Fluor, Chlor und Brom und/oder Nitrogruppen tragen kann.

Die Umsetzung der Verbindungen II mit III, IVa mit V und VI mit VII sind jeweils Veretherungen und werden nach den dafür allgemein bekannten Methoden durchgeführt (Houben-Weyl, Bd. VI/3, S. 1ff, S.49ff (1965)).

Üblicherweise erfolgt die Veretherung bei Temperaturen von -50 bis 150 °C, vorzugsweise von -20 bis 120 °C.

Die Veretherung verläuft im allgemeinen oberhalb von -20 °C mit ausreichender Geschwindigkeit. 120 °C müssen in der Regel für einen vollständigen Umsatz nicht überschritten werden. Die Reaktion kann unter Wärmeentwicklung verlaufen, weshalb es vorteilhaft sein kann, eine Kühlmöglichkeit vorzusehen.

Als Basen kommen allgemein anorganische Verbindungen wie Alkalimetall- und Erdalkalimetallhydroxide, beispielsweise Lithiumhydroxid, Natriumhydroxid, Kaliumhydroxid und Calziumhydroxid, Alkalimetall- und Erdalkalimetalloxide, beispielsweise Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetall- und Erdalkalimetallcarbonate, beispielsweise Lithiumcarbonat und Calziumcarbonat, Alkalimetallhydrogencarbonate, beispielsweise Natriumhydrogencarbonat, metallorganische Verbindungen, insbesondere Alkalimetallalkyle, beispielsweise Methyllithium, Butyllithium und Phenyllithium, Alkalimetall- und Erdalkalimetallalkoholate, beispielsweise Natriummethanolat, Natriumethanolat, Kaliumethanolat, Kalium-tert.-butanolat und Dimethoxymagnesium, außerdem organische Basen, z.B. tertiäre Amine wie Trimethylamin, Triethylamin, Di-isopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Kaliumhydroxid, Kaliumcarbonat, Natriummethylat, Natriumethylat, Kalium-tert.-butylat und Natriumhydrid. Man verwendet im Normalfall mindestens äquivalente Mengen der Base, kann diese aber auch im Überschuß oder gegebenenfalls als Lösungsmittel verwenden.

Als Lösungsmittel eignen sich beispielsweise aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Ethylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol und Isopropanol, sowie aprotisch dipolare Lösungsmittel wie Dimethylsulfoxid, Dimethylformamid und Pyridin, besonders bevorzugt Acetonitril, Ethanol und Dimethylformamid.

Es können auch Gemische der genannten Lösungsmittel verwendet werden.

Die Ausgangsstoffe werden üblicherweise in stöchiometrischen Mengen miteinander umgesetzt. Es kann, beispielsweise zur Steigerung der Ausbeute, vorteilhaft sein, einen der Ausgangsstoffe in einem Überschuß von 0,1 bis 10 mol-Äq., vorzugsweise 0,2 bis 1,5 mol-Äq. zu verwenden.

Sofern $Y^1$ bzw. $Y^2$ in einer der Formeln III, IVa bzw. VII für Chlor oder Brom steht, kann die Umsetzung im allgemeinen durch Zugabe von katalytischen Mengen an Kaliumiodid beschleunigt werden. Der Katalysator wird üblicherweise in Mengen von 5 bis 20 mol-% verwendet.

Die Abspaltung der Schutzgruppe R aus der Verbindung IVb erfolgt in ebenfalls bekannter Weise in einem Lösungsmittel in Gegenwart einer Säure oder eines sauren Katalysators (Houben-Weyl, Bd. VI/1c, S. 314ff (1976)).

Die Ether IVb werden im allgemeinen bei Temperaturen von -20 °C bis 120 °C, vorzugsweise 20 °C bis 100 °C gespalten.

Als Lösungsmittel für die Spaltung eignen sich die im Vorstehenden für die Veretherung genannten, insbesondere Methanol, Ethanol, Chloroform und Dioxan und entsprechende Gemische.

Als Säuren und saure Katalysatoren finden anorganische Säuren wie Fluorwasserstoffsäure, Salzsäure, Bromwasserstoffsäure, Schwefelsäure und Perchlorsäure, Lewis-Säuren wie Bortrifluorid, Aluminiumtrichlorid, Eisen-III-chlorid, Zinn-IV-chlorid, Titan-IV-chlorid und Zink-II-chlorid, sowie organische Säuren wie Ameisensäure, Essigsäure, Propionsäure, Oxalsäure, Zitronensäure und Trifluoressigsäure Verwendung. Die Säuren werden im allgemeinen in katalytischen Mengen eingesetzt, sie können aber auch äquimolar, im Überschuß oder gegebenenfalls als Lösungsmittel verwendet werden.

Man erhält die Diether IVa neben der vorstehend beschriebenen Möglichkeit zur Herstellung aber auch, indem man einen 4-Hydroxyphenolether II in an sich bekannter Weise (US-A 4 310 706) in einem inerten organischen Lösungsmittel in Gegenwart einer Base mit einem cyclischen Carbonat VIII umsetzt und den so erhaltenen Diether-Alkohol IV (Z = Hydroxy) halogeniert oder sulfonyliert.

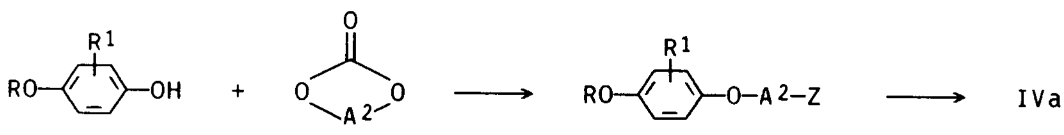

II VIII IV (Z = OH)

Aus den Diether-Alkoholen IV (Z = Hydroxy) erhält man vorteilhaft die Diether IVb, in denen $R^2$ für 2-, 3- oder 4-Pyridyl steht, indem man diese Diether-Alkohole IV (Z = Hydroxy) im Sinne einer nucleophilen Substitution mit einem entsprechenden halogenierten Pyridinderivat IX umsetzt (Houben-Weyl, Bd. V/4, S. 710ff (1960)).

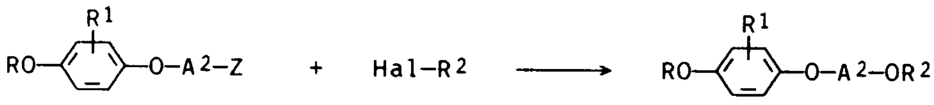

IV (Z = OH) IX IVb

$R^2$ bedeutet in diesem Sinne insbesondere ein Pyridinderivat, welches in 2-, 3- oder 4-Position ein Halogenatom (Hal) wie Fluor, Chlor und Brom trägt. Die Umsetzung verläuft analog zu den vorstehend für die Reaktion von II mit III, IVa mit V bzw. VI mit VII beschriebenen Bedingungen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Umsetzung benötigten Hetarylalkylderivate der Formel VII sind entweder bekannt, kommerziell erhältlich oder lassen sich nach allgemein bekannten chemischen Verfahren herstellen.

Verfahren zur Herstellung von Thiophenderivaten finden sich beispielsweise in: Comprehensive Heterocyclic Chemistry, R. Katritzky und W. Rees, Vol. 4, S. 863 ff, Pergamon Press 1984; Thiazolderivate, Oxazolderivate, Isothiazolderivate, Thiadiazolderivate und Oxadiazolderivate z. B. in: Comprehensive Heterocyclic Chemistry, R. Katritzky und W. Rees, Vol. 6, S. 131, 177, 235, 365, 427, 545 ff, Pergamon Press 1984; Imidazolderivate z. B. in: Advances in Heterocyclic Chemistry, Vol. 27, S. 242 ff, 1980; Pyrazol-und Triazolderivate z. B. in Comprehensive Heterocyclic Chemistry; R. Katritzky und W. Rees, Vol. 5, S. 167, 733 ff, Pergamon Press 1984; Isoxazolderivate z. B. in DE-A-25 49 962 und DE-A-27 54 832.

N-Methylazole sind zum Teil aus Heterocycles 24, 2233-2237 (1986) bekannt oder können nach der dort beschriebenen Methode durch Umsetzung des Azols mit para-Formaldehyd erhalten werden.

Im Hinblick auf die bestimmungsgemäße Verwendung der Verbindungen I in Schädlingsbekämpfungsmitteln kommen als Substituenten folgende Reste in Betracht:

$A^1$

Methylen, Ethylen oder Propylen, vorzugsweise Methylen und Ethylen; insbesondere Methylen, wobei diese Gruppen ein oder zwei $C_1$-$C_3$-Alkylreste wie Methyl, Ethyl, Propyl und 1-Methylethyl, vorzugsweise Methyl und Ethyl insbesondere Methyl tragen können;

$A^2$

Ethylen oder Propylen, wobei diese Gruppen ein oder zwei $C_1$-$C_3$-Alkylreste wie Methyl, Ethyl, Propyl und 1-Methylethyl, vorzugsweise Methyl und Ethyl, insbesondere Methyl tragen können;

$R^1$

Wasserstoff,

Halogen wie Fluor, Chlor, Brom und Iod, vorzugsweise Fluor, Chlor und Brom, insbesondere Fluor und Chlor,

oder $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_3$-Alkyl, insbesondere Methyl;

$R^2$

1-Pyrazolyl, welches ein bis drei der folgenden Reste tragen kann:

Halogen wie Fluor, Chlor, Brom und Iod, vorzugsweise Fluor, Chlor und Brom, insbesondere Fluor und Chlor,

und $C_1$-$C_3$-Alkyl wie Methyl, Ethyl, Propyl und 1-Methylethyl, vorzugsweise Methyl und Ethyl, insbesondere Methyl,

oder 2-, 3- oder 4-Pyridyl, welches ein bis drei der folgenden Reste tragen kann: Cyano, Nitro, Halogen wie Fluor, Chlor, Brom und Iod, vorzugsweise Fluor, Chlor und Brom, insbesondere Fluor und Chlor,

$C_1$-$C_3$-Alkyl wie Methyl, Ethyl, Propyl und 1-Methylethyl, vorzugsweise Methyl und Ethyl, insbesondere Methyl;

$C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl,

2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise $C_1$-Halogenalkyl, insbesondere Trifluormethyl;
$C_1$-$C_3$-Alkoxy wie Methoxy, Ethoxy, Propyloxy und 1-Methylethoxy, vorzugsweise $C_1$-$C_2$-Alkoxy, insbesondere Methoxy;
$C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise $C_1$-Halogenalkoxy, insbesondere Trifluormethoxy;
$C_1$-$C_3$-Alkylthio wie Methylthio, Ethylthio, Propylthio und 1-Methylethylthio, vorzugsweise $C_1$-$C_2$-Alkylthio, insbesondere Methylthio;
$C_1$-$C_3$-Alkoxy-$C_1$-$C_3$-alkyl wie Methoxymethyl, Ethoxymethyl, Propyloxymethyl, 1-Methylethoxymethyl, Methoxy-1-ethyl, Ethoxy-1-ethyl, Propyloxy-1-ethyl, 1-Methylethoxy-1-ethyl, Methoxy-2-ethyl, Ethoxy-2-ethyl, Propyloxy-2-ethyl, 1-Methylethoxy-2-ethyl, Methoxy-1-propyl, Ethoxy-1-propyl, Propyloxy-1-propyl, 1-Methylethoxy-1-propyl, Methoxy-2-propyl, Ethoxy-2-propyl, Propyloxy-2-propyl, 1-Methylethoxy-2-propyl, Methoxy-3-propyl, Ethoxy-3-propyl, Propyloxy-3-propyl, 1-Methylethoxy-3-propyl, vorzugsweise $C_1$-$C_2$-Alkoxy-$C_1$-$C_2$-alkyl, insbesondere Methoxymethyl, Ethoxymethyl und 1-Methoxyethyl,
oder $C_3$-$C_6$-Cycloalkyl wie Cyclopropyl, Cyclobutyl, Cyclopentyl und Cyclohexyl, vorzugsweise Cyclopropyl, Cyclopentyl und Cyclohexyl, insbesondere Cyclopropyl;

X

ein fünfgliedriger Heteroaromat, enthaltend ein bis drei Stickstoffatome und/oder ein Sauerstoff- oder Schwefelatom als Heteroatome wie 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, vorzugsweise ein Heteroaromat, enthaltend ein oder zwei Stickstoffatome oder ein oder zwei Stickstoffatome und ein Sauerstoff- oder ein Schwefelatom, insbesondere 1-Imidazolyl, 3-Isoxazolyl, 5-Isoxazolyl, 1,3,4-Oxadiazol-2-yl und 1,3,4-Thiadiazol-2-yl,
welcher ein bis drei der folgenden Reste tragen kann: Nitro, Halogen wie Fluor, Chlor, Brom und Iod, vorzugsweise Fluor, Chlor und Brom, insbesondere Fluor und Chlor,
$C_1$-$C_6$-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_4$-Alkyl, insbesondere Methyl, Ethyl und Propyl;
$C_1$-$C_4$-Halogenalkyl besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise $C_1$-Halogenalkyl, insbesondere Trifluormethyl;
$C_1$-$C_6$-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Dimethylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethylbutyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, vorzugsweise $C_1$-$C_3$-Alkoxy, insbesondere Methoxy und Ethoxy;
$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise $C_1$-Halogenalkoxy, insbesondere Trifluormethoxy und Difluormethoxy;
$C_1$-$C_4$-Alkylthio wie Methylthio, Ethylthio, Propylthio, 1-Methylethylthio, Butylthio, 1-Methyl-propylthio, 2-Methylpropylthio und 1, 1-Dimethylethylthio, vorzugsweise $C_1$-$C_2$-Alkylthio, insbesondere Methylthio;
$C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise $C_1$-Halogenalkylthio, insbesondere Trifluormethylthio;

$C_1$-$C_3$-Alkoxy-$C_1$-$C_4$-alkyl, besonders $C_1$-$C_3$-Alkoxy-$C_1$-$C_3$-alkyl wie Methoxymethyl, Ethoxymethyl, Propyloxymethyl, 1-Methylethoxymethyl, Methoxy-1-ethyl, Ethoxy-1-ethyl, Propyloxy-1-ethyl, 1-Methylethoxy-1-ethyl, Methoxy-2-ethyl, Ethoxy-2-ethyl, Propyloxy-2-ethyl, 1-Methylethoxy-2-ethyl, Methoxy-1-propyl, Ethoxy-1-propyl, Propyloxy-1-propyl, 1-Methylethoxy-1-propyl, Methoxy-2-propyl, Ethoxy-2-propyl, Propyloxy-2-propyl, 1-Methylethoxy-2-propyl, Methoxy-3-propyl, Ethoxy-3-propyl, Propyloxy-3-propyl, 1-Methylethoxy-3-propyl, vorzugsweise $C_1$-$C_2$-Alkoxy-$C_1$-$C_2$-alkyl, insbesondere Methoxymethyl, Ethoxymethyl und 1-Methoxyethyl;

$C_3$-$C_8$-Cycloalkyl wie Cyclopropyl, Cyclobutyl, Cyclopentyl, Cyclohexyl, Cycloheptyl und Cyclooctyl, vorzugsweise Cyclopropyl, Cyclopentl und Cclohexyl, insbesondere Cyclopropyl;

$C_2$-$C_8$-Alkenyl, besonders $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, vorzugsweise $C_2$-$C_4$-Alkenyl, insbesondere Ethenyl, 1-Propenyl und 2-Propenyl;

Aryl wie Phenyl, 1-Naphthyl und 2-Naphthyl, vorzugsweise Phenyl,

oder durch Aryl wie vorstehend genannt substituiertes $C_1$-$C_{10}$-Alkyl, besonders $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise $C_1$-$C_4$-Alkyl, insbesondere Methyl, Ethyl und Propyl,

wobei die aromatischen Reste ihrerseits ein bis fünf Halogenatome wie Fluor, Chlor, Brom und Iod, vorzugsweise Fluor, Chlor und Brom, insbesondere Fluor und Chlor, und/oder ein bis drei der folgenden Gruppen tragen können:

$C_1$-$C_4$-Alkyl wie Methyl, Ethyl, Propyl, 1-Methylethyl, Butyl, 1-Methylpropyl, 2-Methylpropyl und 1,1-Dimethylethyl, vorzugsweise $C_1$-$C_3$-Alkyl, insbesondere Methyl und Ethyl;

$C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise $C_1$-Halogenalkyl, insbesondere Trifluormethyl;

$C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy, Ethoxy und Propyloxy, insbesondere Methoxy und Ethoxy;

$C_1$-$C_4$-Halogenalkoxy, besonders $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, vorzugsweise $C_1$-Halogenalkoxy, insbesondere Difluormethoxy und Trifluormethoxy;

$C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise $C_1$-$C_2$-Alkylthio, insbesondere Methylthio;

oder $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, vorzugsweise $C_1$-Halogenalkylthio, insbesondere Trifluormethylthio.

Als Substituent X insbesondere bevorzugte 5-gliedrige Heteroaromaten sind nachfolgend zusammengestellt, wobei die möglichen Substituenten der Übersichtlichkeit wegen nicht eingezeichnet sind; die Bindung zu $A^1$ ist als ·- bezeichnet.

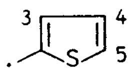

X -1

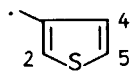

X -2

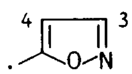

X -3

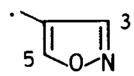

X -4

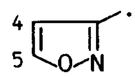

X -5

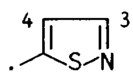

X -6

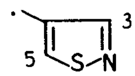

X -7

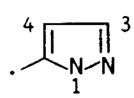

X -8

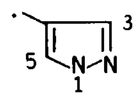

X -9

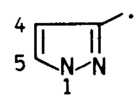

X -10

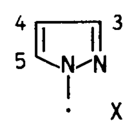

X -11

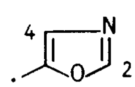

X -12

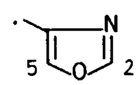

X -13

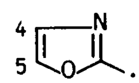

X -14

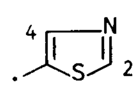

X -15

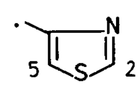

X -16

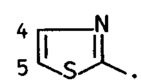

X -17

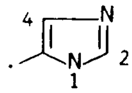
X -18

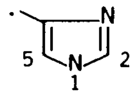
X -19

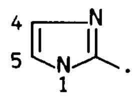
X -20

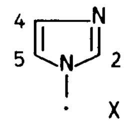
X -21

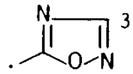
X -22

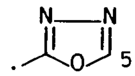
X -23

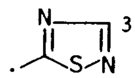
X -24

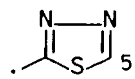
X -25

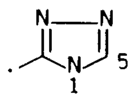
X -26

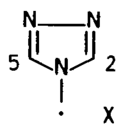
X -27

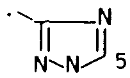
X -28

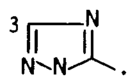
X -29

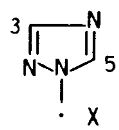
X -30

Beispiele für insbesondere bevorzugte Verbindungen der allgemeinen Formel I sind in den folgenden Tabellen A und B zusammengestellt.

Tabelle A

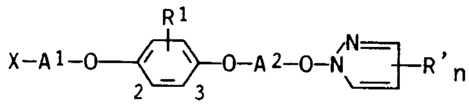

IA

| X | Substituent an X | $A^1$ | $R^1$ | $A^2$ | R'$_n$ |
|---|---|---|---|---|---|
| X-1 | - | $CH_2$ | H | $CH_2CH_2$ | H |
| X-1 | - | $CH(CH_3)$ | H | $CH_2CH_2$ | H |
| X-1 | - | $CH(CH_2CH_3)$ | H | $CH_2CH_2$ | H |
| X-1 | - | $CH_2$ | 3-F | $CH_2CH_2$ | H |
| X-1 | - | $CH_2CH_2$ | H | $CH_2CH_2$ | H |
| X-1 | - | $CH_2$ | H | $CH(CH_3)CH_2$ | H |
| X-1 | - | $CH_2$ | H | $CH_2CH(CH_3)$ | H |
| X-1 | - | $CH_2$ | H | $CH_2CH_2CH_2$ | H |
| X-1 | 5-$CH_3$ | $CH_2$ | H | $CH_2CH_2$ | H |
| X-1 | 4-$CH_3$ | $CH_2$ | H | $CH_2CH_2$ | H |
| X-1 | 3-$CH_3$ | $CH_2$ | H | $CH_2CH_2$ | H |
| X-1 | 5-Cl | $CH_2$ | H | $CH_2CH_2$ | H |
| X-1 | 5-Br | $CH_2$ | H | $CH_2CH_2$ | H |
| X-1 | 4-Cl | $CH_2$ | H | $CH_2CH_2$ | H |
| X-1 | 4-Br | $CH_2$ | H | $CH_2CH_2$ | H |
| X-1 | 5-Cyclopropyl | $CH_2$ | H | $CH_2CH_2$ | H |
| X-1 | 4-Cyclopropyl | $CH_2$ | H | $CH_2CH_2$ | H |
| X-1 | 4,5-Dichlor | $CH_2$ | H | $CH_2CH_2$ | H |
| X-1 | 5-Br | $CH_2$ | H | $CH_2CH_2$ | 4-Cl |
| X-1 | 5-Br | $CH_2$ | H | $CH_2CH_2$ | 3,5-$(CH_3)_2$ |
| X-1 | 4,5-Dichlor | $CH_2$ | 3-F | $CH(CH_3)CH_2$ | 4-Cl |
| X-1 | 5-$CH_3$ | $CH_2$ | H | $CH_2CH(CH_3)$ | 3,5-$(CH_3)_2$ |
| X-1 | 5-Br | $CH_2$ | H | $CH_2CH_2CH_2$ | 4-Cl |
| X-2 | - | $CH_2$ | H | $CH_2CH_2$ | H |
| X-2 | 4-Cyclopropyl | $CH_2$ | H | $CH_2CH_2$ | H |
| X-2 | 5-Cyclopropyl | $CH(CH_3)$ | H | $CH(CH_3)CH_2$ | H |
| X-2 | 4-Cl | $CH_2$ | H | $CH_2CH(CH_3)$ | H |
| X-2 | 5-Br | $CH_2$ | H | $CH_2CH_2$ | H |
| X-2 | 4,5-Dichlor | $CH_2CH_2$ | H | $CH_2CH_2CH_2$ | H |
| X-2 | 4,5-Dibrom | $CH_2$ | 3-F | $CH_2CH_2$ | H |
| X-2 | 5-Br | $CH(CH_2CH_3)$ | H | $CH_2CH_2$ | 3,5-$(CH_3)_2$ |

Tabelle A (Fortsetzung)

| X | Substituent an X | $A^1$ | $R^1$ | $A^2$ | $R'_n$ |
|---|---|---|---|---|---|
| X-2 | 5-Br | $CH_2$ | H | $CH_2CH(CH_3)$ | 4-Cl |
| X-2 | 4-Cl | $CH_2$ | 3-F | $CH(CH_3)CH(CH_3)$ | 4-Cl |
| X-2 | 5-Cyclopropyl | $CH_2$ | H | $CH_2CH(CH_3)$ | $3,5-(CH_3)_2$ |
| X-2 | 4-Cyclopropyl | $CH_2CH_2$ | H | $CH_2CH_2CH_2$ | H |
| X-3 | $3-CH_3$ | $CH_2$ | H | $CH_2CH_2$ | H |
| X-3 | $3-CH_2CH_3$ | $CH_2$ | H | $CH_2CH_2$ | H |
| X-3 | $3-CH(CH_3)_2$ | $CH_2$ | H | $CH_2CH_2$ | H |
| X-3 | 3-Cyclopropyl | $CH_2$ | H | $CH_2CH_2$ | H |
| X-3 | $3-OCH_2CH_3$ | $CH_2$ | H | $CH_2CH_2$ | H |
| X-3 | $3-CH_2OCH_3$ | $CH_2$ | H | $CH_2CH_2$ | H |
| X-3 | $3-CF_3$ | $CH_2$ | H | $CH_2CH_2$ | H |
| X-3 | $3-CH_3$ | $CH_2$ | H | $CH_2CH_2$ | 4-Cl |
| X-3 | 3-Cyclopropyl | $CH_2$ | H | $CH_2CH_2$ | 4-Cl |
| X-3 | $3-CH(CH_3)_2$ | $CH_2$ | H | $CH_2CH(CH_3)$ | $3,5-(CH_3)_2$ |
| X-3 | $3-CH_3$ | $CH_2$ | H | $CH(CH_3)CH_2CH_2$ | H |
| X-3 | 3-Cyclopropyl | $CH_2CH_2CH_2$ | H | $CH_2CH_2$ | 4-Cl |
| X-3 | $3-CF_3$ | $CH_2CH_2$ | 3-F | $CH_2CH_2$ | H |
| X-3 | $3-OCH_2CH_3$ | $CH(CH_3)$ | H | $CH(CH_3)CH(CH_3)$ | H |
| X-3 | $3-CH_2CH_3$ | $CH_2$ | $3-CH_3$ | $CH_2CH_2$ | $3,5-(CH_3)_2$ |
| X-4 | - | $CH_2$ | H | $CH_2CH_2$ | H |
| X-4 | $5-CH_3$ | $CH_2$ | H | $CH_2CH(CH_3)$ | H |
| X-4 | $3-CH_3$ | $CH_2$ | H | $CH_2CH(CH_3)$ | H |
| X-4 | $3-CH(CH_3)_2$ | $CH_2$ | H | $CH_2CH_2$ | 4-Cl |
| X-4 | $3-OCH_2CH_3$ | $CH(CH_3)$ | H | $CH(CH_3)CH_2$ | $3,5-(CH_3)_2$ |
| X-4 | $5-OCH_2CH_3$ | $CH_2$ | 3-F | $CH(CH_3)CH(CH_3)$ | H |
| X-4 | $5-CH(CH_3)_2$ | $CH_2CH_2$ | H | $CH_2CH_2CH(CH_3)$ | H |
| X-5 | - | $CH_2$ | H | $CH_2CH_2$ | H |
| X-5 | $5-CH_3$ | $CH_2$ | H | $CH_2CH_2$ | H |
| X-5 | 5-Cyclopropyl | $CH_2$ | H | $CH_2CH_2$ | H |
| X-5 | $5-OCH_2CH_3$ | $CH_2CH_2$ | H | $CH_2CH(CH_3)$ | 4-Cl |
| X-5 | 5-Cyclopropyl | $CH(CH_3)$ | H | $CH_2CH(CH_3)$ | $3,5-(CH_3)_2$ |
| X-5 | 5-Cyclopropyl | $CH_2$ | H | $CH(CH_3)CH_2$ | H |
| X-5 | $5-CH_3$ | $CH(CH_3)$ | 3-F | $CH_2CH_2$ | 4-Cl |
| X-5 | $5-CH_3$ | $CH_2CH_2CH_2$ | H | $CH_2CH_2CH_2$ | H |
| X-5 | $5-OCH_2CH_3$ | $CH_2$ | H | $CH_2CH(CH_3)$ | $3,5-(CH_3)_2$ |
| X-6 | - | $CH_2$ | 3-F | $CH_2CH_2$ | H |
| X-6 | $3-CH_3$ | $CH_2$ | H | $CH_2CH_2$ | H |
| X-6 | 3-Cyclopropyl | $CH_2$ | H | $CH_2CH_2$ | H |

Tabelle A (Fortsetzung)

| X | Substituent an X | $A^1$ | $R^1$ | $A^2$ | $R'_n$ |
|---|---|---|---|---|---|
| X-6 | 3-$OCH_2CH_3$ | $CH_2$ | H | $CH_2CH_2$ | H |
| X-6 | 3-Cyclopropyl | $CH(CH_3)$ | H | $CH(CH_3)CH_2$ | 4-Cl |
| X-6 | 3-Cyclopropyl | $CH_2CH_2$ | H | $CH_2CH_2CH_2$ | H |
| X-6 | 3-$CH_3$ | $CH_2$ | H | $CH_2CH(CH_3)$ | 4-Cl |
| X-6 | 3-$CH_3$ | $CH_2$ | 3-$CH_3$ | $CH_2CH(CH_3)$ | 3,5-$(CH_3)_2$ |
| X-6 | 3-Cyclopropyl | $CH_2$ | H | $CH(CH_3)CH(CH_3)$ | H |
| X-7 | - | $CH_2$ | H | $CH_2CH_2$ | H |
| X-7 | 3-$CH_3$ | $CH_2$ | H | $CH_2CH_2$ | H |
| X-7 | 3-$CH_3$ | $CH_2$ | 3-$CH_3$ | $CH(CH_3)CH_2$ | H |
| X-7 | 3-$CH_3$ | $CH_2$ | H | $CH(CH_3)CH_2$ | H |
| X-7 | 3-$CH_3$ | $CH(CH_3)$ | H | $CH_2CH(CH_3)$ | 4-Cl |
| X-7 | 3-$CH_3$ | $CH_2$ | H | $CH_2CH(CH_3)$ | 3,5-$(CH_3)_2$ |
| X-8 | 1-$CH_3$ | $CH_2$ | H | $CH_2CH_2$ | H |
| X-8 | 1,3-$(CH_3)_2$ | $CH_2$ | H | $CH_2CH_2$ | H |
| X-8 | 1-$CH_3$, 3-Cyclopropyl | $CH_2$ | H | $CH_2CH_2$ | H |
| X-8 | 1-$CH_3$, 3-Cyclopropyl | $CH(CH_3)$ | H | $CH_2CH_2$ | H |
| X-8 | 1-$CH_3$, 3-Cyclopropyl | $CH_2$ | 3-F | $CH_2CH_2$ | H |
| X-8 | 1,3-$(CH_3)_2$ | $CH_2$ | H | $CH(CH_3)CH(CH_3)$ | 4-Cl |
| X-8 | 1,3-$(CH_3)_2$ | $CH_2$ | H | $CH_2CH(CH_3)$ | 3,5-$(CH_3)_2$ |
| X-8 | 1,4-$(CH_3)_2$ | $CH_2$ | H | $CH_2CH_2$ | H |
| X-8 | 1,4-$(CH_3)_2$ | $CH_2CH_2$ | H | $CH_2CH_2CH_2$ | H |
| X-8 | 1-$CH_3$, 3-Cyclopropyl | $CH_2$ | H | $CH_2CH(CH_3)$ | 4-Cl |
| X-9 | 1-$CH_3$ | $CH_2$ | H | $CH_2CH_2$ | H |
| X-9 | 1,3-$(CH_3)_2$ | $CH_2$ | H | $CH_2CH_2$ | H |
| X-9 | 1,5-$(CH_3)_2$ | $CH_2$ | H | $CH_2CH_2$ | H |
| X-9 | 1,3-$(CH_3)_2$ | $CH_2$ | 3-F | $CH_2CH(CH_3)$ | H |
| X-9 | 1,5-$(CH_3)_2$ | $CH_2$ | 3-$CH_3$ | $CH_2CH(CH_3)$ | H |
| X-9 | 1,3-$(CH_3)_2$ | $CH_2$ | H | $CH_2CH_2$ | 4-Cl |
| X-9 | 1,5-$(CH_3)_2$ | $CH_2$ | H | $CH_2CH_2$ | 3,5-$(CH_3)_2$ |
| X-9 | 1,3-$(CH_3)_2$ | $CH(CH_3)$ | H | $CH(CH_3)CH_2CH(CH_3)$ | H |
| X-9 | 1,5-$(CH_3)_2$ | $CH_2CH_2$ | H | $CH(CH_3)CH_2$ | 4-Cl |
| X-10 | 1-$CH_3$ | $CH_2$ | H | $CH_2CH_2$ | H |
| X-10 | 1,5-$(CH_3)_2$ | $CH_2$ | H | $CH_2CH_2$ | H |
| X-10 | 1-$CH_3$, 5-Cyclopropyl | $CH_2$ | H | $CH_2CH_2$ | H |
| X-10 | 1,4-$(CH_3)_2$ | $CH_2$ | H | $CH_2CH_2$ | H |
| X-10 | 1-$CH_3$, 5-Cyclopropyl | $CH_2$ | H | $CH(CH_3)CH_2$ | 4-Cl |
| X-10 | 1-$CH_3$, 5-Cyclopropyl | $CH_2CH_2$ | H | $CH_2CH_2CH_2$ | H |
| X-10 | 1,5-$(CH_3)_2$ | $CH_2$ | H | $CH_2CH(CH_3)$ | 3,5-$(CH_3)_2$ |

Tabelle A (Fortsetzung)

| X | Substituent an X | $A^1$ | $R^1$ | $A^2$ | $R'_n$ |
|---|---|---|---|---|---|
| X-10 | 1,5-$(CH_3)_2$ | $CH_2$ | H | $CH_2CH_2$ | 4-Cl |
| X-11 | - | $CH_2$ | H | $CH_2CH_2$ | H |
| X-11 | 3-$CH_3$ | $CH_2$ | H | $CH_2CH_2$ | H |
| X-11 | 5-$CH_3$ | $CH_2$ | H | $CH_2CH_2$ | H |
| X-11 | 3-$CH_3$ | $CH_2$ | H | $CH_2CH_2$ | 4-Cl |
| X-11 | 5-$CH_3$ | $CH_2$ | H | $CH_2CH_2$ | 3,5-$(CH_3)_2$ |
| X-11 | 3-$CH_3$ | $CH_2$ | 3-F | $CH(CH_3)CH_2$ | 4-Cl |
| X-11 | 5-$CH_3$ | $CH_2$ | H | $CH_2CH(CH_3)$ | H |
| X-12 | - | $CH_2$ | H | $CH_2CH_2$ | H |
| X-12 | 2-$CH_3$ | $CH_2$ | H | $CH_2CH_2$ | H |
| X-12 | 2-Cyclopropyl | $CH_2$ | H | $CH_2CH_2$ | H |
| X-12 | 2-$OCH_2CH_3$ | $CH_2$ | H | $CH_2CH_2$ | H |
| X-12 | 2-$CH_3$ | $CH_2$ | H | $CH_2CH_2$ | 4-Cl |
| X-12 | 2-Cyclopropyl | $CH_2$ | H | $CH_2CH_2$ | 4-Cl |
| X-12 | 2-$CH_3$ | $CH_2$ | H | $CH_2CH_2$ | 3,5-$(CH_3)_2$ |
| X-12 | 2-Cyclopropyl | $CH_2$ | H | $CH_2CH_2$ | 3,5-$(CH_3)_2$ |
| X-12 | 2-$CH_3$ | $CH(CH_3)$ | H | $CH(CH_3)CH_2CH_2$ | H |
| X-12 | 2-Cyclopropyl | $CH_2$ | H | $CH_2CH(CH_3)$ | 4-Cl |
| X-13 | 2-Cyclopropyl | $CH_2$ | H | $CH_2CH_2$ | H |
| X-13 | 2-$CH_3$ | $CH_2$ | H | $CH_2CH_2$ | H |
| X-13 | 2-Cyclopropyl | $CH_2$ | H | $CH_2CH_2$ | 4-Cl |
| X-13 | 2-$CH_3$ | $CH_2$ | H | $CH_2CH_2$ | 4-Cl |
| X-13 | 2-Cyclopropyl | $CH_2$ | H | $CH_2CH_2$ | 3,5-$(CH_3)_2$ |
| X-13 | 2-$CH_3$ | $CH_2$ | H | $CH_2CH_2$ | 3,5-$(CH_3)_2$ |
| X-13 | 2-$OCH_2CH_3$ | $CH_2$ | H | $CH_2CH_2$ | H |
| X-13 | 2-$CH_3$ | $CH_2$ | H | $CH(CH_3)CH(CH_3)$ | H |
| X-13 | 2-Cyclopropyl | $CH_2$ | H | $CH_2CH(CH_3)$ | 4-Cl |
| X-14 | - | $CH_2$ | H | $CH_2CH_2$ | H |
| X-14 | 5-$CH_3$ | $CH_2$ | H | $CH_2CH_2$ | H |
| X-14 | 5-Cyclopropyl | $CH_2$ | H | $CH_2CH_2$ | H |
| X-14 | 4-$CH_3$ | $CH_2$ | H | $CH_2CH_2$ | 4-Cl |
| X-14 | 4-Cyclopropyl | $CH_2$ | H | $CH_2CH_2$ | 4-Cl |
| X-14 | 5-Cl | $CH_2$ | H | $CH_2CH_2CH(CH_3)$ | H |
| X-14 | 4-$OCH_2CH_3$ | $CH_2$ | H | $CH_2CH_2$ | 3,5-$(CH_3)_2$ |
| X-14 | 5-Cyclopropyl | $CH_2$ | H | $CH(CH_3)CH(CH_3)$ | 4-Cl |
| X-15 | - | $CH_2$ | H | $CH_2CH_2$ | H |
| X-15 | 2-$CH_3$ | $CH_2$ | H | $CH_2CH_2$ | H |
| X-15 | 2-Cyclopropyl | $CH_2$ | H | $CH_2CH_2$ | H |

Tabelle A (Fortsetzung)

| X | Substituent an X | A1 | $R^1$ | $A^2$ | R'n |
|---|---|---|---|---|---|
| X-15 | 2-$OCH_2CH_3$ | $CH_2$ | H | $CH_2CH_2$ | H |
| X-15 | 2-Cl | $CH_2$ | H | $CH_2CH_2$ | H |
| X-15 | 2-$CH_3$ | $CH_2$ | 3-F | $CH_2CH(CH_3)$ | H |
| X-15 | 2-Cyclopropyl | $CH_2CH_2$ | H | $CH(CH_3)CH(CH_3)$ | H |
| X-15 | 2-$CH_3$ | $CH_2$ | H | $CH_2CH_2$ | 4-Cl |
| X-15 | 2-Cyclopropyl | $CH_2$ | H | $CH_2CH_2$ | 4-Cl |
| X-15 | 2-$CH_3$ | $CH_2$ | H | $CH_2CH_2CH_2$ | 3,5-$(CH_3)_2$ |
| X-15 | 2-Cyclopropyl | $CH(CH_3)$ | H | $CH_2CH_2$ | 3,5-$(CH_3)_2$ |
| X-16 | - | $CH_2$ | H | $CH_2CH_2$ | H |
| X-16 | 2-$CH_3$ | $CH_2$ | H | $CH_2CH_2$ | H |
| X-16 | 2-Cyclopropyl | $CH_2$ | H | $CH_2CH_2$ | H |
| X-16 | 2-Cl | $CH_2$ | H | $CH_2CH_2$ | H |
| X-16 | 2-$OCH_2CH_3$ | $CH_2$ | H | $CH_2CH_2$ | H |
| X-16 | 2-$CH_3$ | $CH_2$ | H | $CH_2CH_2$ | 4-Cl |
| X-16 | 2-Cyclopropyl | $CH_2$ | H | $CH_2CH_2$ | 4-Cl |
| X-16 | 2-$CH_3$ | $CH_2$ | H | $CH(CH_3)CH_2$ | 3,5-$(CH_3)_2$ |
| X-16 | 2-Cyclopropyl | $CH_2$ | H | $CH_2CH(CH_3)$ | H |
| X-16 | 2-$CH_3$ | $CH_2CH_2$ | 3-F | $CH_2CH_2CH_2$ | H |
| X-16 | 2-Cyclopropyl | $CH_2$ | H | $CH(CH_3)CH(CH_3)$ | 4-Cl |
| X-17 | - | $CH_2$ | H | $CH_2CH_2$ | H |
| X-17 | 5-Cyclopropyl | $CH_2$ | H | $CH_2CH_2$ | H |
| X-17 | 5-$CH_3$ | $CH_2$ | H | $CH_2CH_2$ | H |
| X-17 | 5-Cl | $CH_2$ | H | $CH_2CH_2$ | H |
| X-17 | 5-$OCH_2CH_3$ | $CH_2$ | H | $CH_2CH_2$ | H |
| X-17 | 4-Cyclopropyl | $CH_2$ | H | $CH_2CH_2$ | H |
| X-17 | 4-$CH_3$ | $CH_2$ | H | $CH_2CH_2$ | H |
| X-17 | 4-Cl | $CH_2$ | H | $CH_2CH_2$ | H |
| X-17 | 4-$OCH_2CH_3$ | $CH_2$ | H | $CH_2CH_2$ | H |
| X-17 | 5-Cyclopropyl | $CH_2$ | H | $CH_2CH_2$ | 4-Cl |
| X-17 | 5-$CH_3$ | $CH(CH_3)$ | 3-$CH_3$ | $CH(CH_3)CH(CH_3)$ | H |
| X-17 | 4-$CH_3$ | $CH_2$ | H | $CH_2CH_2$ | 3,5-$(CH_3)_2$ |
| X-17 | 4-Cyclopropyl | $CH_2CH_2$ | H | $CH_2CH_2CH(CH_3)$ | H |
| X-17 | 5-Cyclopropyl | $CH_2$ | H | $CH_2CH(CH_3)$ | 4-Cl |
| X-18 | 1-$CH_3$ | $CH_2$ | H | $CH_2CH_2$ | H |
| X-18 | 1,2-$(CH_3)_2$ | $CH_2$ | H | $CH_2CH_2$ | H |
| X-18 | 1,4-$(CH_3)_2$ | $CH_2$ | H | $CH_2CH_2$ | H |
| X-18 | 1,2,4-$(CH_3)_3$ | $CH_2$ | H | $CH_2CH_2$ | H |
| X-18 | 1-$CH_3$, 2-Cyclopropyl | $CH_2$ | H | $CH_2CH_2$ | H |

Tabelle A (Fortsetzung)

| X | Substituent an X | $A^1$ | $R^1$ | $A^2$ | R'$_n$ |
|---|---|---|---|---|---|
| X-18 | 1-$CH_3$, 2-Cyclopropyl | $CH_2$ | H | $CH_2CH_2$ | 4-Cl |
| X-18 | 1-$CH_3$, 2-Cyclopropyl | $CH_2$ | H | $CH_2CH_2$ | 3,5-$(CH_3)_2$ |
| X-18 | 1,2-$(CH_3)_2$ | $CH_2$ | H | $CH_2CH(CH_3)$ | 4-Cl |
| X-18 | 1-$CH_3$ | $CH_2CH_2$ | 3-F | $CH_2CH_2CH_2$ | H |
| X-18 | 1,2,4-$(CH_3)_3$ | $CH_2$ | H | $CH(CH_3)CH(CH_3)$ | H |
| X-18 | 1,4-$(CH_3)_2$ | $CH_2$ | H | $CH_2CH(CH_3)$ | 4-Cl |
| X-18 | 1-$CH_3$, 2-Cyclopropyl | $CH(CH_3)$ | H | $CH_2CH(CH_3)$ | 3,5-$(CH_3)_2$ |
| X-18 | 1-$CH_3$, 2-Cyclopropyl | $CH_2$ | H | $CH_2CH_2CH(CH_3)$ | H |
| X-18 | 1-$CH_3$ | $CH_2$ | H | $CH_2CH_2$ | 4-Cl |
| X-19 | 1-$CH_3$ | $CH_2$ | H | $CH_2CH_2$ | H |
| X-19 | 1,2-$(CH_3)_2$ | $CH_2$ | H | $CH_2CH_2$ | H |
| X-19 | 1-$CH_3$, 2-Cyclopropyl | $CH_2$ | H | $CH_2CH_2$ | H |
| X-19 | 1-$CH_3$ | $CH_2$ | H | $CH_2CH_2$ | 4-Cl |
| X-19 | 1,2-$(CH_3)_2$ | $CH_2$ | H | $CH_2CH_2$ | 4-Cl |
| X-19 | 1-$CH_3$, 2-Cyclopropyl | $CH_2$ | H | $CH_2CH_2$ | 4-Cl |
| X-19 | 1-$CH_3$ | $CH_2$ | H | $CH_2CH_2$ | 3,5-$(CH_3)_2$ |
| X-19 | 1,2-$(CH_3)_2$ | $CH_2$ | H | $CH_2CH_2$ | 3,5-$(CH_3)_2$ |
| X-19 | 1-$CH_3$, 2-Cyclopropyl | $CH_2$ | H | $CH_2CH_2$ | 3,5-$(CH_3)_2$ |
| X-19 | 1-$CH_3$ | $CH_2CH_2$ | H | $CH(CH_3)CH_2CH_2$ | H |
| X-19 | 1,2-$(CH_3)_2$ | $CH(CH_3)$ | H | $CH_2CH(CH_3)$ | 4-Cl |
| X-19 | 1-$CH_3$, 2-Cyclopropyl | $CH_2$ | H | $CH(CH_3)CH(CH_3)$ | H |
| X-20 | 1-$CH_3$ | $CH_2$ | H | $CH_2CH_2$ | H |
| X-20 | 1,5-$(CH_3)_2$ | $CH_2$ | H | $CH_2CH_2$ | H |
| X-20 | 1-$CH_3$ | $CH_2$ | H | $CH_2CH_2$ | 4-Cl |
| X-20 | 1,5-$(CH_3)_2$ | $CH_2$ | H | $CH_2CH_2$ | 4-Cl |
| X-20 | 1-$CH_3$ | $CH_2$ | H | $CH_2CH_2CH_2$ | 3,5-$(CH_3)_2$ |
| X-20 | 1,5-$(CH_3)_2$ | $CH_2$ | H | $CH_2CH(CH_3)$ | 4-Cl |
| X-20 | 1-$CH_3$ | $CH(CH_3)$ | H | $CH(CH_3)CH(CH_3)$ | H |
| X-20 | 1,5-$(CH_3)_2$ | $CH_2CH_2$ | H | $CH(CH_3)CH_2$ | H |
| X-21 | - | $CH_2$ | H | $CH_2CH_2$ | H |
| X-21 | 4,5-$Cl_2$ | $CH_2$ | H | $CH_2CH_2$ | H |
| X-21 | 4,5-$Br_2$ | $CH_2$ | H | $CH_2CH_2$ | H |
| X-21 | 4,5-$(CH_3)_2$ | $CH_2$ | H | $CH_2CH_2$ | H |
| X-21 | - | $CH_2$ | H | $CH_2CH_2CH(CH_3)$ | 4-Cl |
| X-21 | 4,5-$Cl_2$ | $CH_2$ | H | $CH_2CH_2$ | 4-Cl |
| X-21 | 4,5-$Br_2$ | $CH(CH_3)$ | H | $CH(CH_3)CH(CH_3)$ | 3,5-$(CH_3)_2$ |
| X-21 | 4,5-$Cl_2$ | $CH_2$ | 3-F | $CH_2CH_2CH(CH_3)$ | H |
| X-22 | - | $CH_2$ | H | $CH_2CH_2$ | H |

Tabelle A (Fortsetzung)

| X | Substituent an X | $A^1$ | $R^1$ | $A^2$ | $R'_n$ |
|---|---|---|---|---|---|
| X-22 | 3-$CH_3$ | $CH_2$ | H | $CH_2CH_2$ | H |
| X-22 | 3-Cyclopropyl | $CH_2$ | H | $CH_2CH_2$ | H |
| X-22 | 3-$OCH_3$ | $CH_2$ | H | $CH_2CH_2$ | H |
| X-22 | 3-$CH_3$ | $CH_2$ | H | $CH_2CH_2$ | 4-Cl |
| X-22 | 3-Cyclopropyl | $CH_2$ | H | $CH_2CH_2$ | 4-Cl |
| X-22 | 3-$CH_3$ | $CH_2$ | H | $CH_2CH_2$ | 3,5-$(CH_3)_2$ |
| X-22 | 3-Cyclopropyl | $CH_2$ | H | $CH_2CH_2$ | 3,5-$(CH_3)_2$ |
| X-22 | 3-$CH_3$ | $CH_2CH_2$ | H | $CH(CH_3)CH_2CH_2$ | H |
| X-22 | 3-$OCH_3$ | $CH_2$ | 3-$CH_3$ | $CH_2CH(CH_3)$ | H |
| X-22 | 3-Cyclopropyl | $CH_2$ | H | $CH(CH_3)CH(CH_3)$ | H |
| X-23 | - | $CH_2$ | H | $CH_2CH_2$ | H |
| X-23 | 5-$CH_3$ | $CH_2$ | H | $CH_2CH_2$ | H |
| X-23 | 5-$CH_2CH_3$ | $CH_2$ | H | $CH_2CH_2$ | H |
| X-23 | 5-$CH(CH_3)_2$ | $CH_2$ | H | $CH_2CH_2$ | H |
| X-23 | 5-Cyclopropyl | $CH_2$ | H | $CH_2CH_2$ | H |
| X-23 | 5-Cl | $CH_2$ | H | $CH_2CH_2$ | H |
| X-23 | 5-$OCH_2CH_3$ | $CH_2$ | H | $CH_2CH_2$ | H |
| X-23 | 5-$CH_3$ | $CH_2$ | H | $CH_2CH_2$ | 4-Cl |
| X-23 | 5-$CH_3$ | $CH_2$ | H | $CH(CH_3)CH(CH_3)$ | 4-Cl |
| X-23 | 5-Cyclopropyl | $CH_2$ | H | $CH_2CH(CH_3)$ | 3,5-$(CH_3)_2$ |
| X-23 | 5-$CH(CH_3)_2$ | $CH_2$ | H | $CH_2CH_2CH_2$ | 4-Cl |
| X-23 | 5-$CH_3$ | $CH(CH_3)$ | 3-$CH_3$ | $CH(CH_3)CH_2CH_2$ | H |
| X-23 | 5-Cyclopropyl | $CH_2$ | H | $CH_2CH_2$ | 3,5-$(CH_3)_2$ |
| X-23 | 5-$CH(CH_3)_2$ | $CH_2$ | 3-F | $CH_2CH_2$ | H |
| X-24 | - | $CH_2$ | H | $CH_2CH_2$ | H |
| X-24 | 3-$CH_3$ | $CH_2$ | H | $CH_2CH_2$ | H |
| X-24 | 3-Cyclopropyl | $CH_2$ | H | $CH_2CH_2$ | H |
| X-24 | 3-$OCH_3$ | $CH_2$ | H | $CH_2CH_2$ | H |
| X-24 | 3-$CH_3$ | $CH_2$ | H | $CH_2CH_2$ | 4-Cl |
| X-24 | 3-Cyclopropyl | $CH_2$ | H | $CH_2CH_2$ | 4-Cl |
| X-24 | 3-$CH_3$ | $CH_2$ | H | $CH_2CH_2$ | 3,5-$(CH_3)_2$ |
| X-24 | 3-Cyclopropyl | $CH_2$ | H | $CH_2CH_2$ | 3,5-$(CH_3)_2$ |
| X-24 | 3-$CH_3$ | $CH_2CH_2$ | H | $CH(CH_3)CH_2CH_2$ | H |
| X-24 | 3-$OCH_3$ | $CH_2$ | 3-F | $CH_2CH(CH_3)$ | H |
| X-24 | 3-Cyclopropyl | $CH_2$ | H | $CH(CH_3)CH(CH_3)$ | H |
| X-25 | 5-$CH_3$ | $CH_2$ | H | $CH_2CH_2$ | H |
| X-25 | 5-$CH_2CH_3$ | $CH_2$ | H | $CH_2CH_2$ | H |
| X-25 | 5-$CH(CH_3)_2$ | $CH_2$ | H | $CH_2CH_2$ | H |

Tabelle A (Fortsetzung)

| X | Substituent an X | $A^1$ | $R^1$ | $A^2$ | $R'_n$ |
|---|---|---|---|---|---|
| X-25 | 5-Cyclopropyl | $CH_2$ | H | $CH_2CH_2$ | H |
| X-25 | 5-$OCH_3$ | $CH_2$ | H | $CH_2CH_2$ | H |
| X-25 | 5-$OCH_2CH_3$ | $CH_2$ | H | $CH_2CH_2$ | H |
| X-25 | 5-Cyclopropyl | $CH_2$ | H | $CH_2CH_2$ | 3,5-$(CH_3)_2$ |
| X-25 | 5-$CH_3$ | $CH_2$ | H | $CH_2CH_2$ | 4-Cl |
| X-25 | 5-$CH(CH_3)_2$ | $CH_2$ | H | $CH_2CH_2$ | 4-Cl |
| X-25 | 5-Cyclopropyl | $CH_2$ | H | $CH_2CH_2$ | 4-Cl |
| X-25 | 5-$CH_3$ | $CH_2$ | H | $CH(CH_3)CH_2$ | H |
| X-25 | 5-Cyclopropyl | $CH_2$ | 3-F | $CH(CH_3)CH_2$ | H |
| X-25 | 5-$OCH_2CH_3$ | $CH_2$ | H | $CH_2CH(CH_3)$ | H |
| X-25 | 5-Cyclopropyl | $CH(CH_3)$ | 3-$CH_3$ | $CH_2CH(CH_3)$ | H |
| X-25 | 5-$CH(CH_3)_2$ | $CH_2$ | H | $CH_2CH(CH_3)$ | 4-Cl |
| X-25 | 5-$CH_3$ | $CH_2$ | H | $CH_2CH(CH_3)$ | 4-Cl |
| X-25 | 5-Cyclopropyl | $CH_2CH_2$ | H | $CH(CH_3)CH(CH_3)$ | 3,5-$(CH_3)_2$ |
| X-25 | 5-Cyclopropyl | $CH_2$ | H | $CH_2CH_2CH_2$ | H |
| X-26 | 1-$CH_3$ | $CH_2$ | H | $CH_2CH_2$ | H |
| X-26 | 1,5-$(CH_3)_2$ | $CH_2$ | H | $CH_2CH_2$ | H |
| X-26 | 1-$CH_3$, 5-Cyclopropyl | $CH_2$ | H | $CH_2CH_2$ | H |
| X-26 | 1-$CH_3$ | $CH_2$ | H | $CH(CH_3)CH(CH_3)$ | 4-Cl |
| X-26 | 1,5-$(CH_3)_2$ | $CH_2$ | H | $CH_2CH_2CH(CH_3)$ | 4-Cl |
| X-26 | 1-$CH_3$, 5-Cyclopropyl | $CH_2$ | H | $CH_2CH_2$ | 3,5-$(CH_3)_2$ |
| X-26 | 1-$CH_3$ | $CH(CH_3)$ | H | $CH_2CH_2CH_2$ | H |
| X-26 | 1-$CH_3$, 5-Cyclopropyl | $CH_2CH_2$ | 3-F | $CH_2CH_2$ | 4-Cl |
| X-27 | - | $CH_2$ | H | $CH_2CH_2$ | H |
| X-27 | 2-Cl | $CH_2$ | H | $CH_2CH_2$ | H |
| X-27 | 2,5-$(CH_3)_2$ | $CH_2$ | H | $CH_2CH_2$ | H |
| X-27 | - | $CH_2$ | H | $CH_2CH_2$ | 4-Cl |
| X-27 | 2-Cl | $CH_2$ | H | $CH_2CH_2$ | 4-Cl |
| X-27 | 2,5-$(CH_3)_2$ | $CH_2$ | H | $CH_2CH_2$ | 4-Cl |
| X-27 | - | $CH(CH_3)$ | 3-F | $CH(CH_3)CH_2CH_2$ | H |
| X-27 | - | $CH_2$ | H | $CH_2CH(CH_3)$ | 3,5-$(CH_3)_2$ |
| X-28 | 1-$CH_3$ | $CH_2$ | H | $CH_2CH_2$ | H |
| X-28 | 1,5-$(CH_3)_2$ | $CH_2$ | H | $CH_2CH_2$ | H |
| X-28 | 1-$CH_3$, 5-Cyclopropyl | $CH_2$ | H | $CH_2CH_2$ | H |
| X-28 | 1-$CH_3$ | $CH_2$ | H | $CH_2CH_2$ | 4-Cl |
| X-28 | 1,5-$(CH_3)_2$ | $CH_2$ | H | $CH_2CH_2$ | 4-Cl |
| X-28 | 1-$CH_3$, 5-Cyclopropyl | $CH_2$ | H | $CH_2CH_2$ | 4-Cl |
| X-28 | 1-$CH_3$ | $CH_2$ | H | $CH_2CH(CH_3)$ | 3,5-$(CH_3)_2$ |

Tabelle A (Fortsetzung)

| X | Substituent an X | $A^1$ | $R^1$ | $A^2$ | $R'_n$ |
|---|---|---|---|---|---|
| X-28 | 1,5-$(CH_3)_2$ | $CH_2$ | H | $CH_2CH(CH_3)$ | 3,5-$(CH_3)_2$ |
| X-28 | 1-$CH_3$, 5-Cyclopropyl | $CH_2$ | H | $CH_2CH(CH_3)$ | 3,5-$(CH_3)_2$ |
| X-28 | 1-$CH_3$ | $CH_2CH_2$ | H | $CH_2CH_2CH_2$ | H |
| X-29 | 1-$CH_3$ | $CH_2$ | H | $CH_2CH_2$ | H |
| X-29 | 1,3-$(CH_3)_2$ | $CH_2$ | H | $CH_2CH_2$ | H |
| X-29 | 1-$CH_3$, 3-Cyclopropyl | $CH_2$ | H | $CH_2CH_2$ | H |
| X-29 | 1-$CH_3$ | $CH_2$ | H | $CH_2CH_2$ | 4-Cl |
| X-29 | 1,3-$(CH_3)_2$ | $CH_2$ | H | $CH_2CH_2$ | 4-Cl |
| X-29 | 1-$CH_3$, 3-Cyclopropyl | $CH_2$ | H | $CH_2CH_2$ | 4-Cl |
| X-29 | 1-$CH_3$ | $CH_2$ | H | $CH_2CH_2CH_2$ | 3,5-$(CH_3)_2$ |
| X-30 | - | $CH_2$ | H | $CH_2CH_2$ | H |
| X-30 | 3,5-$(CH_3)_2$ | $CH_2$ | H | $CH_2CH_2$ | H |
| X-30 | - | $CH_2$ | H | $CH_2CH_2$ | 4-Cl |
| X-30 | 3,5-$(CH_3)_2$ | $CH_2$ | H | $CH_2CH_2$ | 4-Cl |
| X-30 | - | $CH_2$ | 3-F | $CH(CH_3)CH_2$ | H |
| X-30 | 3,5-$(CH_3)_2$ | $CH(CH_3)$ | H | $CH_2CH(CH_3)$ | H |
| X-30 | - | $CH_2$ | H | $CH_2CH(CH_3)$ | 3,5-$(CH_3)_2$ |
| X-30 | 3,5-$(CH_3)_2$ | $CH_2CH_2$ | H | $CH_2CH_2CH_2$ | H |

Tabelle B

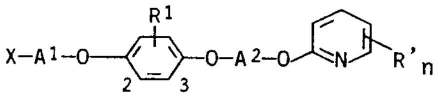

IB

| X | Substituent an X | $A^1$ | $R^1$ | $A^2$ | R'n |
|---|---|---|---|---|---|
| X-1 | - | $CH_2$ | H | $CH_2CH_2$ | H |
| X-1 | - | $CH(CH_3)$ | H | $CH_2CH_2$ | H |
| X-1 | - | $CH(CH_2CH_3)$ | H | $CH_2CH_2$ | H |
| X-1 | - | $CH_2$ | 3-F | $CH_2CH_2$ | H |
| X-1 | - | $CH_2CH_2$ | H | $CH_2CH_2$ | H |
| X-1 | - | $CH_2$ | H | $CH(CH_3)CH_2$ | H |
| X-1 | - | $CH_2$ | H | $CH_2CH(CH_3)$ | H |
| X-1 | - | $CH_2$ | H | $CH_2CH_2CH(CH_3)$ | H |
| X-1 | 5-$CH_3$ | $CH_2$ | H | $CH_2CH_2$ | H |
| X-1 | 4-$CH_3$ | $CH_2$ | H | $CH_2CH_2$ | H |
| X-1 | 3-$CH_3$ | $CH_2$ | H | $CH_2CH_2$ | H |
| X-1 | 5-Cl | $CH_2$ | H | $CH_2CH(CH_3)$ | H |
| X-1 | 5-Br | $CH_2$ | H | $CH_2CH(CH_3)$ | H |
| X-1 | 4-Cl | $CH_2$ | H | $CH_2CH(CH_3)$ | H |
| X-1 | 4-Br | $CH_2$ | H | $CH_2CH(CH_3)$ | H |
| X-1 | 5-Cyclopropyl | $CH_2$ | H | $CH_2CH_2$ | H |
| X-1 | 4-Cyclopropyl | $CH_2$ | H | $CH_2CH_2$ | H |
| X-1 | 4,5-Dichlor | $CH_2$ | H | $CH_2CH(CH_3)$ | H |
| X-1 | 5-Br | $CH_2$ | H | $CH_2CH(CH_3)$ | 6-$CH_3$ |
| X-1 | 5-Br | $CH_2$ | H | $CH_2CH(CH_3)$ | 5-$NO_2$ |
| X-1 | 4,5-Dichlor | $CH_2$ | 3-F | $CH(CH_3)CH_2$ | H |
| X-1 | 5-$CH_3$ | $CH_2$ | H | $CH_2CH(CH_3)$ | 6-$CH_3$ |
| X-1 | 5-Br | $CH_2$ | H | $CH_2CH(CH_3)$ | 5-$NO_2$ |
| X-2 | - | $CH_2$ | H | $CH_2CH_2$ | H |
| X-2 | 4-Cyclopropyl | $CH_2$ | H | $CH_2CH(CH_3)$ | H |
| X-2 | 5-Cyclopropyl | $CH(CH_3)$ | H | $CH(CH_3)CH_2$ | H |
| X-2 | 4-Cl | $CH_2$ | H | $CH_2CH(CH_3)$ | H |
| X-2 | 5-Br | $CH_2$ | H | $CH_2CH(CH_3)$ | H |
| X-2 | 4,5-Dichlor | $CH_2CH_2$ | H | $CH_2CH_2$ | H |
| X-2 | 4,5-Dibrom | $CH_2$ | 3-F | $CH_2CH(CH_3)$ | H |
| X-2 | 5-Br | $CH(CH_2CH_3)$ | H | $CH_2CH(CH_3)$ | H |
| X-2 | 5-Br | $CH_2$ | H | $CH_2CH_2$ | 6-$CH_3$ |
| X-2 | 4-Cl | $CH_2$ | 3-F | $CH(CH_3)CH(CH_3)$ | 5-$NO_2$ |
| X-2 | 5-Cyclopropyl | $CH_2$ | H | $CH_2CH(CH_3)$ | 6-$CH_3$ |
| X-2 | 4-Cyclopropyl | $CH_2CH_2$ | H | $CH_2CH_2CH_2$ | H |

Tabelle B (Fortsetzung)

| X | Substituent an X | $A^1$ | $R^1$ | $A^2$ | $R'_n$ |
|---|---|---|---|---|---|
| X-3 | 3-$CH_3$ | $CH_2$ | H | $CH_2CH(CH_3)$ | H |
| X-3 | 3-$CH_2CH_3$ | $CH_2$ | H | $CH_2CH(CH_3)$ | H |
| X-3 | 3-$CH(CH_3)_2$ | $CH_2$ | H | $CH_2CH(CH_3)$ | H |
| X-3 | 3-Cyclopropyl | $CH_2$ | H | $CH_2CH(CH_3)$ | H |
| X-3 | 3-$OCH_2CH_3$ | $CH_2$ | H | $CH_2CH(CH_3)$ | H |
| X-3 | 3-$CH_2OCH_3$ | $CH_2$ | H | $CH_2CH(CH_3)$ | H |
| X-3 | 3-$CF_3$ | $CH_2$ | H | $CH_2CH(CH_3)$ | H |
| X-3 | 3-$CH_3$ | $CH_2$ | H | $CH_2CH_2$ | 6-$CH_3$ |
| X-3 | 3-Cyclopropyl | $CH_2$ | H | $CH_2CH_2$ | 5-$NO_2$ |
| X-3 | 3-$CH(CH_3)_2$ | $CH_2$ | H | $CH_2CH(CH_3)$ | 6-$CH_3$ |
| X-3 | 3-$CH_3$ | $CH_2$ | H | $CH(CH_3)CH_2CH_2$ | H |
| X-3 | 3-Cyclopropyl | $CH_2CH_2CH_2$ | H | $CH_2CH(CH_3)$ | H |
| X-3 | 3-$CF_3$ | $CH_2CH_2$ | 3-F | $CH_2CH(CH_3)$ | 6-$CH_3$ |
| X-3 | 3-$OCH_2CH_3$ | $CH(CH_3)$ | H | $CH(CH_3)CH(CH_3)$ | H |
| X-3 | 3-$CH_2CH_3$ | $CH_2$ | 3-$CH_3$ | $CH_2CH_2$ | 5-$NO_2$ |
| X-4 | - | $CH_2$ | H | $CH_2CH_2$ | H |
| X-4 | 5-$CH_3$ | $CH_2$ | H | $CH_2CH(CH_3)$ | H |
| X-4 | 3-$CH_3$ | $CH_2$ | H | $CH_2CH(CH_3)$ | H |
| X-4 | 3-$CH(CH_3)_2$ | $CH_2$ | H | $CH_2CH(CH_3)$ | H |
| X-4 | 3-$OCH_2CH_3$ | $CH(CH_3)$ | H | $CH(CH_3)CH_2$ | 6-$CH_3$ |
| X-4 | 5-$OCH_2CH_3$ | $CH_2$ | 3-F | $CH(CH_3)CH_2$ | 5-$NO_2$ |
| X-4 | 5-$CH(CH_3)_2$ | $CH_2CH_2$ | H | $CH_2CH_2CH_2$ | H |
| X-5 | - | $CH_2$ | H | $CH_2CH(CH_3)$ | H |
| X-5 | 5-$CH_3$ | $CH_2$ | H | $CH_2CH(CH_3)$ | H |
| X-5 | 5-Cyclopropyl | $CH_2$ | H | $CH_2CH(CH_3)$ | H |
| X-5 | 5-$OCH_2CH_3$ | $CH_2CH_2$ | H | $CH_2CH(CH_3)$ | H |
| X-5 | 5-Cyclopropyl | $CH(CH_3)$ | H | $CH_2CH_2$ | 6-$CH_3$ |
| X-5 | 5-Cyclopropyl | $CH_2$ | H | $CH(CH_3)CH_2$ | 5-$NO_2$ |
| X-5 | 5-$CH_3$ | $CH(CH_3)$ | 3-F | $CH_2CH(CH_3)$ | H |
| X-5 | 5-$CH_3$ | $CH_2CH_2CH_2$ | H | $CH_2CH_2CH_2$ | H |
| X-5 | 5-$OCH_2CH_3$ | $CH_2$ | H | $CH_2CH(CH_3)$ | 6-$CH_3$ |
| X-6 | - | $CH_2$ | 3-F | $CH_2CH(CH_3)$ | H |
| X-6 | 3-$CH_3$ | $CH_2$ | H | $CH_2CH(CH_3)$ | H |
| X-6 | 3-Cyclopropyl | $CH_2$ | H | $CH_2CH(CH_3)$ | H |
| X-6 | 3-$OCH_2CH_3$ | $CH_2$ | H | $CH_2CH_2$ | H |
| X-6 | 3-Cyclopropyl | $CH(CH_3)$ | H | $CH(CH_3)CH_2$ | H |
| X-6 | 3-Cyclopropyl | $CH_2CH_2$ | H | $CH_2CH(CH_3)$ | 6-$CH_3$ |
| X-6 | 3-$CH_3$ | $CH_2$ | H | $CH_2CH_2CH_2$ | 5-$NO_2$ |

Tabelle B (Fortsetzung)

| X | Substituent an X | $A^1$ | $R^1$ | $A^2$ | R'n |
|---|---|---|---|---|---|
| X-6 | 3-$CH_3$ | $CH_2$ | 3-$CH_3$ | $CH_2CH_2$ | H |
| X-6 | 3-Cyclopropyl | $CH_2$ | H | $CH(CH_3)CH(CH_3)$ | H |
| X-7 | - | $CH_2$ | H | $CH_2CH(CH_3)$ | H |
| X-7 | 3-$CH_3$ | $CH_2$ | H | $CH_2CH(CH_3)$ | H |
| X-7 | 3-$CH_3$ | $CH_2$ | 3-$CH_3$ | $CH(CH_3)CH_2$ | H |
| X-7 | 3-$CH_3$ | $CH_2$ | H | $CH(CH_3)CH_2CH(CH_3)$ | H |
| X-7 | 3-$CH_3$ | $CH(CH_3)$ | H | $CH_2CH_2$ | 6-$CH_3$ |
| X-7 | 3-$CH_3$ | $CH_2$ | H | $CH_2CH(CH_3)$ | 6-$CH_3$ |
| X-8 | 1-$CH_3$ | $CH_2$ | H | $CH_2CH(CH_3)$ | H |
| X-8 | 1,3-$(CH_3)_2$ | $CH_2$ | H | $CH_2CH(CH_3)$ | H |
| X-8 | 1-$CH_3$, 3-Cyclopropyl | $CH_2$ | H | $CH_2CH(CH_3)$ | H |
| X-8 | 1-$CH_3$, 3-Cyclopropyl | $CH(CH_3)$ | H | $CH_2CH_2$ | H |
| X-8 | 1-$CH_3$, 3-Cyclopropyl | $CH_2$ | 3-F | $CH_2CH(CH_3)$ | H |
| X-8 | 1,3-$(CH_3)_2$ | $CH_2$ | H | $CH(CH_3)CH(CH_3)$ | 6-$CH_3$ |
| X-8 | 1,3-$(CH_3)_2$ | $CH_2$ | H | $CH_2CH_2CH_2$ | 5-$NO_2$ |
| X-8 | 1,4-$(CH_3)_2$ | $CH_2$ | H | $CH_2CH(CH_3)$ | H |
| X-8 | 1,4-$(CH_3)_2$ | $CH_2CH_2$ | H | $CH_2CH_2CH_2$ | H |
| X-8 | 1-$CH_3$, 3-Cyclopropyl | $CH_2$ | H | $CH_2CH(CH_3)$ | 6-$CH_3$ |
| X-9 | 1-$CH_3$ | $CH_2$ | H | $CH_2CH(CH_3)$ | H |
| X-9 | 1,3-$(CH_3)_2$ | $CH_2$ | H | $CH_2CH(CH_3)$ | H |
| X-9 | 1,5-$(CH_3)_2$ | $CH_2$ | H | $CH_2CH(CH_3)$ | H |
| X-9 | 1,3-$(CH_3)_2$ | $CH_2$ | 3-F | $CH_2CH(CH_3)$ | 6-$CH_3$ |
| X-9 | 1,5-$(CH_3)_2$ | $CH_2$ | 3-$CH_3$ | $CH_2CH(CH_3)$ | 5-$NO_2$ |
| X-9 | 1,3-$(CH_3)_2$ | $CH_2$ | H | $CH_2CH_2$ | H |
| X-9 | 1,5-$(CH_3)_2$ | $CH_2$ | H | $CH_2CH_2$ | H |
| X-9 | 1,3-$(CH_3)_2$ | $CH(CH_3)$ | H | $CH(CH_3)CH(CH_3)$ | H |
| X-9 | 1,5-$(CH_3)_2$ | $CH_2CH_2$ | H | $CH(CH_3)CH(CH_3)$ | H |
| X-10 | 1-$CH_3$ | $CH_2$ | H | $CH_2CH(CH_3)$ | H |
| X-10 | 1,5-$(CH_3)_2$ | $CH_2$ | H | $CH_2CH(CH_3)$ | H |
| X-10 | 1-$CH_3$, 5-Cyclopropyl | $CH_2$ | H | $CH_2CH(CH_3)$ | H |
| X-10 | 1,4-$(CH_3)_2$ | $CH_2$ | H | $CH_2CH(CH_3)$ | H |
| X-10 | 1-$CH_3$, 5-Cyclopropyl | $CH_2$ | H | $CH(CH_3)CH_2$ | 6-$CH_3$ |
| X-10 | 1-$CH_3$, 5-Cyclopropyl | $CH_2CH_2$ | H | $CH_2CH_2$ | 5-$NO_2$ |
| X-10 | 1,5-$(CH_3)_2$ | $CH_2$ | H | $CH_2CH(CH_3)$ | 6-$CH_3$ |
| X-10 | 1,5-$(CH_3)_2$ | $CH_2$ | H | $CH_2CH_2CH_2$ | 6-$CH_3$ |
| X-11 | - | $CH_2$ | H | $CH_2CH(CH_3)$ | H |
| X-11 | 3-$CH_3$ | $CH_2$ | H | $CH_2CH(CH_3)$ | H |
| X-11 | 5-$CH_3$ | $CH_2$ | H | $CH_2CH(CH_3)$ | H |

Tabelle B (Fortsetzung)

| X | Substituent an X | $A^1$ | $R^1$ | $A^2$ | $R'_n$ |
|---|---|---|---|---|---|
| X-11 | 3-$CH_3$ | $CH_2$ | H | $CH_2CH_2$ | H |
| X-11 | 5-$CH_3$ | $CH_2$ | H | $CH_2CH_2$ | H |
| X-11 | 3-$CH_3$ | $CH_2$ | 3-F | $CH(CH_3)CH_2$ | 6-$CH_3$ |
| X-11 | 5-$CH_3$ | $CH_2$ | H | $CH_2CH(CH_3)$ | 5-$NO_2$ |
| X-12 | - | $CH_2$ | H | $CH_2CH(CH_3)$ | H |
| X-12 | 2-$CH_3$ | $CH_2$ | H | $CH_2CH(CH_3)$ | H |
| X-12 | 2-Cyclopropyl | $CH_2$ | H | $CH_2CH(CH_3)$ | H |
| X-12 | 2-$OCH_2CH_3$ | $CH_2$ | H | $CH_2CH(CH_3)$ | H |
| X-12 | 2-$CH_3$ | $CH_2$ | H | $CH_2CH_2$ | H |
| X-12 | 2-Cyclopropyl | $CH_2$ | H | $CH_2CH_2$ | H |
| X-12 | 2-$CH_3$ | $CH_2$ | H | $CH_2CH(CH_3)$ | 6-$CH_3$ |
| X-12 | 2-Cyclopropyl | $CH_2$ | H | $CH_2CH(CH_3)$ | 6-$CH_3$ |
| X-12 | 2-$CH_3$ | $CH(CH_3)$ | H | $CH(CH_3)CH_2$ | 5-$NO_2$ |
| X-12 | 2-Cyclopropyl | $CH_2$ | H | $CH_2CH(CH_3)$ | 5-$NO_2$ |
| X-13 | 2-Cyclopropyl | $CH_2$ | H | $CH_2CH(CH_3)$ | H |
| X-13 | 2-$CH_3$ | $CH_2$ | H | $CH_2CH(CH_3)$ | H |
| X-13 | 2-Cyclopropyl | $CH_2$ | H | $CH_2CH_2$ | H |
| X-13 | 2-$CH_3$ | $CH_2$ | H | $CH_2CH_2$ | H |
| X-13 | 2-Cyclopropyl | $CH_2$ | H | $CH_2CH(CH_3)$ | 6-$CH_3$ |
| X-13 | 2-$CH_3$ | $CH_2$ | H | $CH_2CH(CH_3)$ | 5-$NO_2$ |
| X-13 | 2-$OCH_2CH_3$ | $CH_2$ | H | $CH_2CH(CH_3)$ | H |
| X-13 | 2-$CH_3$ | $CH_2$ | H | $CH(CH_3)CH_2$ | H |
| X-13 | 2-Cyclopropyl | $CH_2$ | H | $CH_2CH_2CH_2$ | 6-$CH_3$ |
| X-14 | - | $CH_2$ | H | $CH_2CH(CH_3)$ | H |
| X-14 | 5-$CH_3$ | $CH_2$ | H | $CH_2CH(CH_3)$ | H |
| X-14 | 5-Cyclopropyl | $CH_2$ | H | $CH_2CH(CH_3)$ | H |
| X-14 | 4-$CH_3$ | $CH_2$ | H | $CH_2CH(CH_3)$ | H |
| X-14 | 4-Cyclopropyl | $CH_2$ | H | $CH_2CH(CH_3)$ | H |
| X-14 | 5-Cl | $CH_2$ | H | $CH_2CH(CH_3)$ | H |
| X-14 | 4-$OCH_2CH_3$ | $CH_2$ | H | $CH_2CH(CH_3)$ | H |
| X-14 | 5-Cyclopropyl | $CH_2$ | H | $CH(CH_3)CH_2$ | 6-$CH_3$ |
| X-15 | - | $CH_2$ | H | $CH_2CH(CH_3)$ | H |
| X-15 | 2-$CH_3$ | $CH_2$ | H | $CH_2CH(CH_3)$ | H |
| X-15 | 2-Cyclopropyl | $CH_2$ | H | $CH_2CH(CH_3)$ | H |
| X-15 | 2-$OCH_2CH_3$ | $CH_2$ | H | $CH_2CH(CH_3)$ | H |
| X-15 | 2-Cl | $CH_2$ | H | $CH_2CH(CH_3)$ | H |
| X-15 | 2-$CH_3$ | $CH_2$ | 3-F | $CH_2CH_2$ | 5-$NO_2$ |
| X-15 | 2-Cyclopropyl | $CH_2CH_2$ | H | $CH(CH_3)CH_2CH_2$ | H |

Tabelle B (Fortsetzung)

| X | Substituent an X | $A^1$ | $R^1$ | $A^2$ | $R'_n$ |
|---|---|---|---|---|---|
| X-15 | 2-$CH_3$ | $CH_2$ | H | $CH_2CH_2$ | 6-$CH_3$ |
| X-15 | 2-Cyclopropyl | $CH_2$ | H | $CH_2CH_2$ | 6-$CH_3$ |
| X-15 | 2-$CH_3$ | $CH_2$ | H | $CH_2CH(CH_3)$ | 5-$NO_2$ |
| X-15 | 2-Cyclopropyl | $CH(CH_3)$ | H | $CH_2CH(CH_3)$ | H |
| X-16 | - | $CH_2$ | H | $CH_2CH(CH_3)$ | H |
| X-16 | 2-$CH_3$ | $CH_2$ | H | $CH_2CH(CH_3)$ | H |
| X-16 | 2-Cyclopropyl | $CH_2$ | H | $CH_2CH(CH_3)$ | H |
| X-16 | 2-Cl | $CH_2$ | H | $CH_2CH(CH_3)$ | H |
| X-16 | 2-$OCH_2CH_3$ | $CH_2$ | H | $CH_2CH(CH_3)$ | H |
| X-16 | 2-$CH_3$ | $CH_2$ | H | $CH_2CH_2$ | 6-$CH_3$ |
| X-16 | 2-Cyclopropyl | $CH_2$ | H | $CH_2CH_2$ | 6-$CH_3$ |
| X-16 | 2-$CH_3$ | $CH_2$ | H | $CH(CH_3)CH_2CH_2$ | H |
| X-16 | 2-Cyclopropyl | $CH_2$ | H | $CH_2CH(CH_3)$ | 5-$NO_2$ |
| X-16 | 2-$CH_3$ | $CH_2CH_2$ | 3-F | $CH_2CH_2CH_2$ | H |
| X-16 | 2-Cyclopropyl | $CH_2$ | H | $CH(CH_3)CH(CH_3)$ | H |
| X-17 | - | $CH_2$ | H | $CH_2CH(CH_3)$ | H |
| X-17 | 5-Cyclopropyl | $CH_2$ | H | $CH_2CH(CH_3)$ | H |
| X-17 | 5-$CH_3$ | $CH_2$ | H | $CH_2CH(CH_3)$ | H |
| X-17 | 5-Cl | $CH_2$ | H | $CH_2CH(CH_3)$ | H |
| X-17 | 5-$OCH_2CH_3$ | $CH_2$ | H | $CH_2CH(CH_3)$ | H |
| X-17 | 4-Cyclopropyl | $CH_2$ | H | $CH_2CH(CH_3)$ | H |
| X-17 | 4-$CH_3$ | $CH_2$ | H | $CH_2CH(CH_3)$ | H |
| X-17 | 4-Cl | $CH_2$ | H | $CH_2CH(CH_3)$ | H |
| X-17 | 4-$OCH_2CH_3$ | $CH_2$ | H | $CH_2CH(CH_3)$ | H |
| X-17 | 5-Cyclopropyl | $CH_2$ | H | $CH_2CH(CH_3)$ | 6-$CH_3$ |
| X-17 | 5-$CH_3$ | $CH(CH_3)$ | 3-$CH_3$ | $CH(CH_3)CH_2$ | H |
| X-17 | 4-$CH_3$ | $CH_2$ | H | $CH_2CH_2$ | 5-$NO_2$ |
| X-17 | 4-Cyclopropyl | $CH_2CH_2$ | H | $CH_2CH_2CH_2$ | H |
| X-17 | 5-Cyclopropyl | $CH_2$ | H | $CH_2CH(CH_3)$ | 5-$NO_2$ |
| X-18 | 1-$CH_3$ | $CH_2$ | H | $CH_2CH(CH_3)$ | H |
| X-18 | 1,2-$(CH_3)_2$ | $CH_2$ | H | $CH_2CH(CH_3)$ | H |
| X-18 | 1,4-$(CH_3)_2$ | $CH_2$ | H | $CH_2CH(CH_3)$ | H |
| X-18 | 1,2,4-$(CH_3)_3$ | $CH_2$ | H | $CH_2CH(CH_3)$ | H |
| X-18 | 1-$CH_3$, 2-Cyclopropyl | $CH_2$ | H | $CH_2CH(CH_3)$ | H |
| X-18 | 1-$CH_3$, 2-Cyclopropyl | $CH_2$ | H | $CH_2CH(CH_3)$ | 6-$CH_3$ |
| X-18 | 1-$CH_3$, 2-Cyclopropyl | $CH_2$ | H | $CH_2CH(CH_3)$ | 5-$NO_2$ |
| X-18 | 1,2-$(CH_3)_2$ | $CH_2$ | H | $CH_2CH_2$ | H |
| X-18 | 1-$CH_3$ | $CH_2CH_2$ | 3-F | $CH_2CH_2CH_2$ | H |

Tabelle B (Fortsetzung)

| X | Substituent an X | $A^1$ | $R^1$ | $A^2$ | $R'_n$ |
|---|---|---|---|---|---|
| X-18 | $1,2,4-(CH_3)_3$ | $CH_2$ | H | $CH(CH_3)CH(CH_3)$ | H |
| X-18 | $1,4-(CH_3)_2$ | $CH_2$ | H | $CH_2CH_2$ | $6-CH_3$ |
| X-18 | $1-CH_3$, 2-Cyclopropyl | $CH(CH_3)$ | H | $CH_2CH(CH_3)$ | H |
| X-18 | $1-CH_3$, 2-Cyclopropyl | $CH_2$ | H | $CH_2CH_2$ | $5-NO_2$ |
| X-18 | $1-CH_3$ | $CH_2$ | H | $CH_2CH(CH_3)$ | $6-CH_3$ |
| X-19 | $1-CH_3$ | $CH_2$ | H | $CH_2CH(CH_3)$ | H |
| X-19 | $1,2-(CH_3)_2$ | $CH_2$ | H | $CH_2CH(CH_3)$ | H |
| X-19 | $1-CH_3$, 2-Cyclopropyl | $CH_2$ | H | $CH_2CH(CH_3)$ | H |
| X-19 | $1-CH_3$ | $CH_2$ | H | $CH_2CH(CH_3)$ | $6-CH_3$ |
| X-19 | $1,2-(CH_3)_2$ | $CH_2$ | H | $CH_2CH(CH_3)$ | $6-CH_3$ |
| X-19 | $1-CH_3$, 2-Cyclopropyl | $CH_2$ | H | $CH_2CH(CH_3)$ | $6-CH_3$ |
| X-19 | $1-CH_3$ | $CH_2$ | H | $CH_2CH_2$ | H |
| X-19 | $1,2-(CH_3)_2$ | $CH_2$ | H | $CH_2CH_2$ | H |
| X-19 | $1-CH_3$, 2-Cyclopropyl | $CH_2$ | H | $CH_2CH_2$ | H |
| X-19 | $1-CH_3$ | $CH_2CH_2$ | H | $CH(CH_3)CH_2CH_2$ | $5-NO_2$ |
| X-19 | $1,2-(CH_3)_2$ | $CH(CH_3)$ | H | $CH_2CH(CH_3)$ | $5-NO_2$ |
| X-19 | $1-CH_3$, 2-Cyclopropyl | $CH_2$ | H | $CH(CH_3)CH(CH_3)$ | H |
| X-20 | $1-CH_3$ | $CH_2$ | H | $CH_2CH(CH_3)$ | H |
| X-20 | $1,5-(CH_3)_2$ | $CH_2$ | H | $CH_2CH(CH_3)$ | H |
| X-20 | $1-CH_3$ | $CH_2$ | H | $CH_2CH_2$ | H |
| X-20 | $1,5-(CH_3)_2$ | $CH_2$ | H | $CH_2CH_2$ | H |
| X-20 | $1-CH_3$ | $CH_2$ | H | $CH_2CH(CH_3)$ | $6-CH_3$ |
| X-20 | $1,5-(CH_3)_2$ | $CH_2$ | H | $CH_2CH(CH_3)$ | $5-NO_2$ |
| X-20 | $1-CH_3$ | $CH(CH_3)$ | H | $CH(CH_3)CH_2CH(CH_3)$ | H |
| X-20 | $1,5-(CH_3)_2$ | $CH_2CH_2$ | H | $CH(CH_3)CH_2$ | H |
| X-21 | - | $CH_2$ | H | $CH_2CH(CH_3)$ | H |
| X-21 | $4,5-Cl_2$ | $CH_2$ | H | $CH_2CH(CH_3)$ | H |
| X-21 | $4,5-Br_2$ | $CH_2$ | H | $CH_2CH(CH_3)$ | H |
| X-21 | $4,5-(CH_3)_2$ | $CH_2$ | H | $CH_2CH(CH_3)$ | H |
| X-21 | - | $CH_2$ | H | $CH_2CH(CH_3)$ | $6-CH_3$ |
| X-21 | $4,5-Cl_2$ | $CH_2$ | H | $CH_2CH(CH_3)$ | $6-CH_3$ |
| X-21 | $4,5-Br_2$ | $CH(CH_3)$ | H | $CH(CH_3)CH_2CH_2$ | H |
| X-21 | $4,5-Cl_2$ | $CH_2$ | 3-F | $CH_2CH_2$ | $5-NO_2$ |
| X-22 | - | $CH_2$ | H | $CH_2CH(CH_3)$ | H |
| X-22 | $3-CH_3$ | $CH_2$ | H | $CH_2CH(CH_3)$ | H |
| X-22 | 3-Cyclopropyl | $CH_2$ | H | $CH_2CH(CH_3)$ | H |
| X-22 | $3-OCH_3$ | $CH_2$ | H | $CH_2CH(CH_3)$ | H |
| X-22 | $3-CH_3$ | $CH_2$ | H | $CH_2CH_2$ | H |

Tabelle B (Fortsetzung)

| X | Substituent an X | $A^1$ | $R^1$ | $A^2$ | $R'_n$ |
|---|---|---|---|---|---|
| X-22 | 3-Cyclopropyl | $CH_2$ | H | $CH_2CH_2$ | H |
| X-22 | 3-$CH_3$ | $CH_2$ | H | $CH_2CH(CH_3)$ | 6-$CH_3$ |
| X-22 | 3-Cyclopropyl | $CH_2$ | H | $CH_2CH(CH_3)$ | 5-$NO_2$ |
| X-22 | 3-$CH_3$ | $CH_2CH_2$ | H | $CH(CH_3)CH(CH_3)$ | H |
| X-22 | 3-$OCH_3$ | $CH_2$ | 3-$CH_3$ | $CH_2CH_2CH_2$ | H |
| X-22 | 3-Cyclopropyl | $CH_2$ | H | $CH(CH_3)CH(CH_3)$ | 6-$CH_3$ |
| X-23 | - | $CH_2$ | H | $CH_2CH(CH_3)$ | H |
| X-23 | 5-$CH_3$ | $CH_2$ | H | $CH_2CH(CH_3)$ | H |
| X-23 | 5-$CH_2CH_3$ | $CH_2$ | H | $CH_2CH(CH_3)$ | H |
| X-23 | 5-$CH(CH_3)_2$ | $CH_2$ | H | $CH_2CH(CH_3)$ | H |
| X-23 | 5-Cyclopropyl | $CH_2$ | H | $CH_2CH(CH_3)$ | H |
| X-23 | 5-Cl | $CH_2$ | H | $CH_2CH(CH_3)$ | H |
| X-23 | 5-$OCH_2CH_3$ | $CH_2$ | H | $CH_2CH(CH_3)$ | H |
| X-23 | 5-$CH_3$ | $CH_2$ | H | $CH_2CH(CH_3)$ | 6-$CH_3$ |
| X-23 | 5-$CH_3$ | $CH_2$ | H | $CH(CH_3)CH_2$ | H |
| X-23 | 5-Cyclopropyl | $CH_2$ | H | $CH_2CH_2$ | H |
| X-23 | 5-$CH(CH_3)_2$ | $CH_2$ | H | $CH_2CH_2$ | H |
| X-23 | 5-$CH_3$ | $CH(CH_3)$ | 3-$CH_3$ | $CH(CH_3)CH_2$ | H |
| X-23 | 5-Cyclopropyl | $CH_2$ | H | $CH_2CH_2CH(CH_3)$ | H |
| X-23 | 5-$CH(CH_3)_2$ | $CH_2$ | 3-F | $CH_2CH(CH_3)$ | H |
| X-24 | - | $CH_2$ | H | $CH_2CH(CH_3)$ | H |
| X-24 | 3-$CH_3$ | $CH_2$ | H | $CH_2CH(CH_3)$ | H |
| X-24 | 3-Cyclopropyl | $CH_2$ | H | $CH_2CH(CH_3)$ | H |
| X-24 | 3-$OCH_3$ | $CH_2$ | H | $CH_2CH(CH_3)$ | H |
| X-24 | 3-$CH_3$ | $CH_2$ | H | $CH_2CH_2$ | H |
| X-24 | 3-Cyclopropyl | $CH_2$ | H | $CH_2CH_2$ | H |
| X-24 | 3-$CH_3$ | $CH_2$ | H | $CH_2CH(CH_3)$ | 6-$CH_3$ |
| X-24 | 3-Cyclopropyl | $CH_2$ | H | $CH_2CH(CH_3)$ | 6-$CH_3$ |
| X-24 | 3-$CH_3$ | $CH_2CH_2$ | H | $CH(CH_3)CH_2CH_2$ | H |
| X-24 | 3-$OCH_3$ | $CH_2$ | 3-F | $CH_2CH_2$ | 5-$NO_2$ |
| X-24 | 3-Cyclopropyl | $CH_2$ | H | $CH(CH_3)CH_2CH(CH_3)$ | H |
| X-25 | 5-$CH_3$ | $CH_2$ | H | $CH_2CH(CH_3)$ | H |
| X-25 | 5-$CH_2CH_3$ | $CH_2$ | H | $CH_2CH(CH_3)$ | H |
| X-25 | 5-$CH(CH_3)_2$ | $CH_2$ | H | $CH_2CH(CH_3)$ | H |
| X-25 | 5-Cyclopropyl | $CH_2$ | H | $CH_2CH(CH_3)$ | H |
| X-25 | 5-$OCH_3$ | $CH_2$ | H | $CH_2CH(CH_3)$ | H |
| X-25 | 5-$OCH_2CH_3$ | $CH_2$ | H | $CH_2CH(CH_3)$ | H |
| X-25 | 5-Cyclopropyl | $CH_2$ | H | $CH_2CH_2$ | H |

Tabelle B (Fortsetzung)

| X | Substituent an X | $A^1$ | $R^1$ | $A^2$ | $R'_n$ |
|---|---|---|---|---|---|
| X-25 | 5-$CH_3$ | $CH_2$ | H | $CH_2CH_2$ | H |
| X-25 | 5-$CH(CH_3)_2$ | $CH_2$ | H | $CH_2CH_2$ | H |
| X-25 | 5-Cyclopropyl | $CH_2$ | H | $CH_2CH(CH_3)$ | 6-$CH_3$ |
| X-25 | 5-$CH_3$ | $CH_2$ | H | $CH(CH_3)CH_2$ | H |
| X-25 | 5-Cyclopropyl | $CH_2$ | 3-F | $CH(CH_3)CH_2$ | H |
| X-25 | 5-$OCH_2CH_3$ | $CH_2$ | H | $CH_2CH(CH_3)$ | 6-$CH_3$ |
| X-25 | 5-Cyclopropyl | $CH(CH_3)$ | 3-$CH_3$ | $CH_2CH_2$ | H |
| X-25 | 5-$CH(CH_3)_2$ | $CH_2$ | H | $CH_2CH_2CH_2$ | H |
| X-25 | 5-$CH_3$ | $CH_2$ | H | $CH_2CH(CH_3)$ | 5-$NO_2$ |
| X-25 | 5-Cyclopropyl | $CH_2CH_2$ | H | $CH(CH_3)CH(CH_3)$ | H |
| X-25 | 5-Cyclopropyl | $CH_2$ | H | $CH_2CH_2CH_2$ | H |
| X-26 | 1-$CH_3$ | $CH_2$ | H | $CH_2CH(CH_3)$ | H |
| X-26 | 1,5-$(CH_3)_2$ | $CH_2$ | H | $CH_2CH(CH_3)$ | H |
| X-26 | 1-$CH_3$, 5-Cyclopropyl | $CH_2$ | H | $CH_2CH(CH_3)$ | H |
| X-26 | 1-$CH_3$ | $CH_2$ | H | $CH(CH_3)CH_2$ | H |
| X-26 | 1,5-$(CH_3)_2$ | $CH_2$ | H | $CH_2CH_2$ | 6-$CH_3$ |
| X-26 | 1-$CH_3$, 5-Cyclopropyl | $CH_2$ | H | $CH_2CH_2CH_2$ | H |
| X-26 | 1-$CH_3$ | $CH(CH_3)$ | H | $CH_2CH(CH_3)$ | H |
| X-26 | 1-$CH_3$, 5-Cyclopropyl | $CH_2CH_2$ | 3-F | $CH_2CH_2$ | 5-$NO_2$ |
| X-27 | - | $CH_2$ | H | $CH_2CH(CH_3)$ | H |
| X-27 | 2-Cl | $CH_2$ | H | $CH_2CH(CH_3)$ | H |
| X-27 | 2,5-$(CH_3)_2$ | $CH_2$ | H | $CH_2CH(CH_3)$ | H |
| X-27 | - | $CH_2$ | H | $CH_2CH_2$ | H |
| X-27 | 2-Cl | $CH_2$ | H | $CH_2CH_2$ | H |
| X-27 | 2,5-$(CH_3)_2$ | $CH_2$ | H | $CH_2CH_2$ | 6-$CH_3$ |
| X-27 | - | $CH(CH_3)$ | 3-F | $CH(CH_3)CH(CH_3)$ | H |
| X-27 | - | $CH_2$ | H | $CH_2CH(CH_3)$ | 5-$NO_2$ |
| X-28 | 1-$CH_3$ | $CH_2$ | H | $CH_2CH(CH_3)$ | H |
| X-28 | 1,5-$(CH_3)_2$ | $CH_2$ | H | $CH_2CH(CH_3)$ | H |
| X-28 | 1-$CH_3$, 5-Cyclopropyl | $CH_2$ | H | $CH_2CH(CH_3)$ | H |
| X-28 | 1-$CH_3$ | $CH_2$ | H | $CH_2CH_2$ | H |
| X-28 | 1,5-$(CH_3)_2$ | $CH_2$ | H | $CH_2CH_2$ | H |
| X-28 | 1-$CH_3$, 5-Cyclopropyl | $CH_2$ | H | $CH_2CH_2$ | H |
| X-28 | 1-$CH_3$ | $CH_2$ | H | $CH_2CH(CH_3)$ | 6-$CH_3$ |
| X-28 | 1,5-$(CH_3)_2$ | $CH_2$ | H | $CH_2CH(CH_3)$ | 5-$NO_2$ |
| X-28 | 1-$CH_3$, 5-Cyclopropyl | $CH_2$ | H | $CH_2CH(CH_3)$ | 6-$CH_3$ |
| X-28 | 1-$CH_3$ | $CH_2CH_2$ | H | $CH_2CH_2CH_2$ | H |
| X-29 | 1-$CH_3$ | $CH_2$ | H | $CH_2CH(CH_3)$ | H |

Tabelle B (Fortsetzung)

| X | Substituent an X | $A^1$ | $R^1$ | $A^2$ | $R'_n$ |
|---|---|---|---|---|---|
| X-29 | 1,3-$(CH_3)_2$ | $CH_2$ | H | $CH_2CH(CH_3)$ | H |
| X-29 | 1-$CH_3$, 3-Cyclopropyl | $CH_2$ | H | $CH_2CH(CH_3)$ | H |
| X-29 | 1-$CH_3$ | $CH_2$ | H | $CH_2CH_2$ | H |
| X-29 | 1,3-$(CH_3)_2$ | $CH_2$ | H | $CH_2CH_2$ | 6-$CH_3$ |
| X-29 | 1-$CH_3$, 3-Cyclopropyl | $CH_2$ | H | $CH_2CH(CH_3)$ | 6-$CH_3$ |
| X-29 | 1-$CH_3$ | $CH_2$ | H | $CH_2CH(CH_3)$ | 5-$NO_2$ |
| X-30 | - | $CH_2$ | H | $CH_2CH(CH_3)$ | H |
| X-30 | 3,5-$(CH_3)_2$ | $CH_2$ | H | $CH_2CH(CH_3)$ | H |
| X-30 | - | $CH_2$ | H | $CH_2CH_2$ | H |
| X-30 | 3,5-$(CH_3)_2$ | $CH_2$ | H | $CH_2CH_2$ | H |
| X-30 | - | $CH_2$ | 3-F | $CH(CH_3)CH_2$ | 6-$CH_3$ |
| X-30 | 3,5-$(CH_3)_2$ | $CH(CH_3)$ | H | $CH_2CH_2CH_2$ | H |
| X-30 | - | $CH_2$ | H | $CH_2CH(CH_3)$ | 6-$CH_3$ |
| X-30 | 3,5-$(CH_3)_2$ | $CH_2CH_2$ | H | $CH_2CH(CH_3)$ | 5-$NO_2$ |

Im Hinblick auf ihre Verwendung als Zwischenprodukte zur Synthese der Hydrochinondiether I werden Diether der allgemeinen Formel IV Verwendet, in der die Substituenten folgende Bedeutung haben:

$A^2$ und $R^1$ im allgemeinen und im besonderen die vorsehend genannten Reste;

R

$C_1$-$C_6$-Alkyl wie vorstehend genannt, vorzugsweise $C_1$-$C_4$-Alkyl, insbesondere 1,1-Dimethylethyl;

Benzyl, wobei der aromatische Rest ein bis fünf Halogenatome wie vorstehend genannt, vorzugsweise Fluor, Chlor und Brom, insbesondere Fluor und Chlor,

und/oder ein bis drei der folgenden Gruppen tragen kann: Cyano, Nitro,

$C_1$-$C_4$-Alkyl wie vorstehend genannt, vorzugsweise Methyl, Ethyl und Propyl, insbesondere Methyl;

$C_1$-$C_4$-Alkoxy wie vorstehend genannt, vorzugsweise $C_1$-$C_2$-Alkoxy, insbesondere Methoxy;

oder $C_1$-$C_4$-Alkylthio wie vorstehend genannt, vorzugsweise $C_1$-$C_2$-Alkylthio, insbesondere Methylthio,

Silyl, welches drei der folgenden Reste trägt: $C_1$-$C_6$-Alkyl wie vorstehend genannt, vorzugsweise $C_1$-$C_4$-Alkyl, insbesondere Methyl, Ethyl und 1-Methylethyl;

und/oder Phenyl, wobei der aromatische Rest ein bis fünf Halogenatome wie vorstehend genannt, vorzugsweise Fluor, Chlor und Brom, insbesondere Fluor und Chlor,

und/oder ein bis drei der folgenden Gruppen tragen kann: Cyano, Nitro,

$C_1$-$C_4$-Alkyl wie vorstehend genannt, vorzugsweise $C_1$-$C_2$-Alkyl, insbesondere Methyl;

$C_1$-$C_4$-Alkoxy wie vorstehend genannt, vorzugsweise $C_1$-$C_2$-Alkoxy, insbesondere Methoxy;

oder $C_1$-$C_4$-Alkylthio wie vorstehend genannt, vorzugsweise $C_1$-$C_2$-Alkylthio, insbesondere Methylthio,

Z

Hydroxy; Halogen wie vorstehend genannt, vorzugsweise Chlor und Brom, insbesondere Brom,

Sulfonyl, welches eine der folgenden Gruppen trägt:

$C_1$-$C_{10}$-Alkyl, besonders $C_1$-$C_6$-Alkyl wie vorstehend genannt, vorzugsweise $C_1$-$C_4$-Alkyl, insbesondere Methyl,

oder Phenyl, wobei der aromatische Rest ein bis fünf Halogenatome wie vorstehend genannt, vorzugsweise Fluor, Chlor und Brom, insbesondere Fluor und Chlor,

und/oder ein bis drei der folgenden Gruppen tragen kann: Cyano, Nitro,

$C_1$-$C_4$-Alkyl wie vorstehend genannt, vorzugsweise $C_1$-$C_2$-Alkyl, insbesondere Methyl;

$C_1$-$C_4$-Alkoxy wie vorstehend genannt, vorzugsweise $C_1$-$C_2$-Alkoxy; insbesondere Methoxy;

oder $C_1$-$C_4$-Alkylthio wie vorstehend genannt, vorzugsweise $C_1$-$C_2$-Alkylthio, insbesondere Methylthio,

oder eine der im allgemeinen und im besonderen bei $R^2$ genannten Gruppen.

Im Hinblick auf ihre Verwendung als Zwischenprodukte zur Synthese der Hydrochinondiether I werden außerdem Monoether der allgemeinen Formel VI verwendet, in der die Substituenten $R^1$, $R^2$ und $A^2$ im allgemeinen und im besonderen die vorsehend genannte Bedeutung haben.

Die Verbindungen der Formel I sind geeignet, Schädlinge aus der Klasse der Insekten, Spinnentiere und Nematoden wirksam zu bekämpfen. Sie können im Pflanzenschutz sowie auf dem Hygiene-,

Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grndiosella, Earias insulana, Elasmopalpus lignosellus, Eupoecilia ambiguella, Evetria bouliana, Feltia subterranea, Galleria mellonella, Grapholita funebrana, Grapholita molesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flamea, Pectinophora gossypiella, Peridroma saucia, Phalera bucephala, Phthorimaea operculella, Phyllocnistis citrella, Pieris brassicae, Plathypena scarbra, Plutella xylostella, Pseudoplusia includens, Phyacionia frustrana, Scrobipalpula absoluta, Sitotroga cerelella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Onlema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossia morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euchistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dyasphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus birittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobins megnini, Paratetranychus pilosus, Permanyssus gallinae, Phyllocaptrata oleivora, Polyphagotarsonemus latus, Psoroptes ovis, Rhipicephalus appendiculatus, Rhipicephalus evertsi, Saccoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycinae, Heterodera schatii, Hetrodera triflolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:

I. 5 Gew.-Teile der Verbindung Nr. 1.019 werden mit 95 Gew.-Teilen feinteiligem Kaolin innig vermischt. Man erhält auf diese Weise ein Stäubemittel, das 5 Gew.% des Wirkstoffs enthält.

II. 30 Gew.-Teile der Verbindung Nr. 1.007 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).

III. 10 Gew.-Teile der Verbindung Nr. 1.024 werden in einer Mischung gelöst, die aus 90 Gew.-Teilen

Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).

IV. 20 Gew.-Teile der Verbindung Nr. 1.013 werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).

V. 80 Gew.-Teile der Verbindung Nr. 1.009 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).

VI. Man vermischt 90 Gew.-Teile der Verbindung Nr. 1.021 mit 10 Gew.-Teilen N-Methyl-α-pyrrolidon und erhält eine Lösung, die zur Anwendung in Form kleinster Tropfen geeignet ist (Wirkstoffgehalt 90 Gew.%).

VII. 20 Gew.-Teile der Verbindung Nr. 1.003 werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.

VIII. 20 Gew.-Teile des Wirkstoffs Nr. 1.001 werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff beträgt unter Freilandbedingungen 0,01 bis 3, vorzugsweise 0,05 bis 1 kg/ha aktive Substanz (a.S.).

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der nachstehenden Tabelle mit physikalischen Angaben aufgeführt.

1. N-2-[4-(1,1-dimethylethoxy)-phenoxy-]ethoxy-pyrazol

Eine Suspension von 4,95 g (1,1 eq.) NaH (80%ige Dispersion in Mineralöl) in 40 ml DMF wurde bei RT tropfenweise mit einer Lösung von 12,7 g (0,15 mol) N-Hydroxypyrazol in 20 ml DMF versetzt und die Mischung 1 h auf 70° C erwärmt. Nach Zugabe einer Lösung von 40,95 g (0,15 mol) 2-[4-(1,1-Dimethylethoxy)-phenoxy]ethylbromid in 100 ml DMF wurde 15 h auf 125° C erhitzt. Das Lösungsmittel wurde danach am Rotationsverdampfer abgezogen, der Rückstand in Ethylacetat aufgenommen und je zweimal mit 5 %iger NaOH-Lösung und Wasser gewaschen. Nach Trocknung und Entfernung des Lösungsmittels im

Vakuum wurden 38,4 g (93 %) der gewünschten Verbindung als helles Öl erhalten, welches ohne weitere Reinigung in die nächste Stufe eingesetzt wurde.
$^1$H-NMR (250 MHz, $CDCl_3$): $\delta$ = 7,38 (d, 1H); 7,28 (d, 1H); 6,93 (d, 2H); 6,83 (d, 2H); 6,17 (t, 1H); 4,63 (m, 2H); 4,17 (m, 2H); 1,30 (s, 9H) ppm

2. N-2-(4-Hydroxyphenoxy-)ethoxy-pyrazol

Eine Lösung von 38,4 g (139 mmol) N-2-[4-(1,1-dimethylethoxy)-phenoxy]ethoxy-pyrazol in 400 ml Ethanol/Wasser (10/1) wurde mit 9 ml konzentrierter Salzsäure versetzt und anschließend 8 h auf Rückfluß erhitzt. Nach Abkühlung auf RT wurde das Lösungsmittel im Vakuum entfernt, der Rückstand in Ethylacetat aufgenommen und mehrmals mit Wasser gewaschen. Nach Trocknung und Einengen der organischen Phase wurden 29,6 g (97 %) der gewünschten Verbindung als zähes Öl erhalten, welches ohne weitere Reinigung in die nächste Stufe eingesetzt wurde.
$^1$H-NMR (250 MHz, $CDCl_3$): $\delta$ = 7,38 (d, 1H); 7,28 (d, 1H); 6,80 (s, 4H); 6,18 (t, 1H); 5,8 (br. s, 1H); 4,62 (m, 2H); 4,12 (m, 2H) ppm

3. 1-(3-Cyclopropyl-isoxazol-5-yl-methoxy-)-4-[2-(1-pyrazolyloxy-)ethoxy]-benzol (Verb. Nr. 1.003)

Eine Suspension von 0,37 g (1,1 eq.) NaH (80%ige Dispersion in Mineralöl) in 20 ml DMF wurde bei RT tropfenweise mit einer Lösung von 2,5 g (11,4 mmol) N-2(4-hydroxyphenoxy-)ethoxy-pyrazol in 30 ml DMF versetzt und die Mischung 1 h auf 70 °C erwärmt. Nach Zugabe einer Lösung von 1,8 g (11,4 mmol) 3-Cyclopropyl-5-chlormethyl-isoxazol in 20 ml DMF wurde 12 h auf 125 °C erhitzt. Das Lösungsmittel wurde eingeengt, der Rückstand in Ethylacetat aufgenommen und je dreimal mit gesättigter $NaHCO_3$-Lösung und Wasser gewaschen. Nach Trocknung, Einengen des Lösungsmittels und chromatographischer Reinigung des Rohprodukts (Kieselgel; Cyclohexan/Ethylacetat = 1/2) wurden 2,3 g (60 %) des gewünschten Hydrochinondiethers erhalten; Fp.: 65-67 °C.

4. 1-(2-Methyl-1,3,4-thiadiazol-5-yl-methoxy)-4-[2-(1-pyrazolyloxy-) ethoxy-]benzol (Verbindung Nr. 1.009)

Eine Suspension von 0,55 g (1,1 eq.) NaH (80%ige Dispersion in Mineralöl) in 30 ml DMF wurde bei RT tropfenweise mit einer Lösung von 3,7 g (16,8 mmol) N-2(4-Hydroxyphenoxy-)ethoxy-pyrazol in 30 ml DMF versetzt und die Mischung 1 h auf 70 °C erwärmt. Nach Zugabe einer Lösung von 2,5 g (16,8 mmol) 2-Methyl-5-chlormethyl-1,3,4-thiadiazol in 20 ml DMF wurde 8 h auf 120 °C erhitzt. Nach Abkühlung auf RT wurde die Mischung mit Ethylacetat verdünnt und je zweimal mit 5%iger NaOH-Lösung, Wasser und gesättigter NaCl-Lösung gewaschen. Einengen des Lösungsmittels und Umkristallisation des erhaltenen Feststoffs aus Hexan/Ethylacetat (4/1) ergab 3,1 g (56 %) des gewünschten Hydrochinondiethers; Fp.: 93-96 °C.

5. 1-(2-Methyl-1,3,4-thiadiazol-5-yl-methoxy)-4-[2-methyl-2-(2-pyridinyloxy)ethoxy-]benzol (Verb. Nr. 1.021)

Eine Suspension von 0,6 g (1,1 eq.) NaH (80%ige Dispersion in Mineralöl) in 30 ml DMF wurde bei RT mit einer Lösung von 4,46 g (18,2 mmol) 4-[2-Methyl-2(2-pyridinyloxy-)ethoxy]-phenol in 30 ml DMF versetzt und 1 h auf 80 °C erwärmt. Nach Zugabe einer Lösung von 2,7 g (18,2 mmol) 2-Methyl-5-chlormethyl-1,3,4-thiadiazol in 20 ml DMF wurde 6 h auf 120 °C erhitzt. Das Lösungsmittel wurde am Rotationsverdampfer abgezogen, der Rückstand in Ethylacetat aufgenommen und dreimal mit Wasser gewaschen. Nach Trocknung, Einengen des Lösungsmittels und chromatographischer Reinigung des Rohprodukts (Kieselgel ; Cyclohexan/Ethylacetat = 2/1) wurden 2,9 g (45 %) farblose Kristalle erhalten; Fp.: 72-78 °C.

Tabelle 1

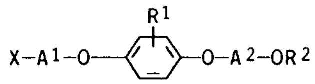

I

| Nr. | X | $A^1$ | $R^1$ | $A^2$ | $R^2$ | physik. Daten Fp. [°C] $^1H$-NMR (Resonanzsignal von $A^1$) [ppm] ($CDCl_3$, 250 MHz) |
|---|---|---|---|---|---|---|
| 1.001 | 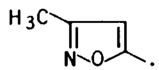 | $CH_2$ | H | $CH_2CH_2$ | 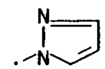 | 82-84 |
| 1.002 | 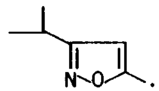 | $CH_2$ | H | $CH_2CH_2$ | 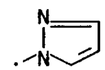 | δ = 5,14 |
| 1.003 | 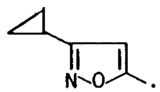 | $CH_2$ | H | $CH_2CH_2$ | 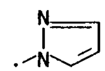 | 65-67 |
| 1.004 | 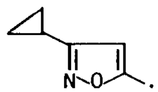 | $CH_2$ | H | $CH_2CH_2$ | 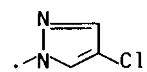 | 90-91 |
| 1.005 | 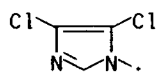 | $CH_2$ | H | $CH_2CH_2$ | 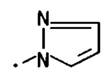 | 82-88 |
| 1.006 | 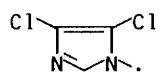 | $CH_2$ | H | $CH_2CH_2$ | 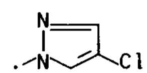 | 66-72 |

Tabelle 1 (Fortsetzung)

| Nr. | X | $A^1$ | $R^1$ | $A^2$ | $R^2$ | physik. Daten Fp. [°C] $^1H$-NMR (Resonanzsignal von $A^1$) [ppm] ($CDCl_3$, 250 MHz) |
|---|---|---|---|---|---|---|
| 1.007 | 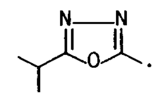 | $CH_2$ | H | $CH_2CH_2$ | 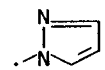 | δ = 5,17 |
| 1.008 | 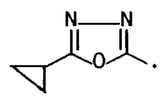 | $CH_2$ | H | $CH_2CH_2$ | 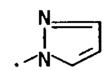 | 47-48 |
| 1.009 | 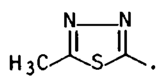 | $CH_2$ | H | $CH_2CH_2$ | 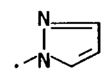 | 93-96 |
| 1.010 | 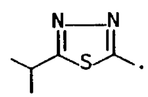 | $CH_2$ | H | $CH_2CH_2$ | 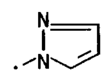 | δ = 5,38 |
| 1.011 | 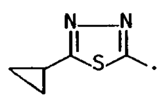 | $CH_2$ | H | $CH_2CH_2$ | 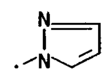 | 64-65 |
| 1.012 | 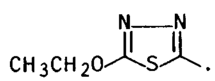 | $CH_2$ | H | $CH_2CH_2$ | 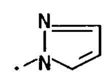 | δ = 4,98 |
| 1.013 | 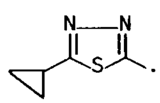 | $CH_2$ | H | $CH_2CH_2$ | 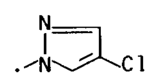 | 55-59 |

Tabelle 1 (Fortsetzung)

| Nr. | X | $A^1$ | $R^1$ | $A^2$ | $R^2$ | physik. Daten Fp. [°C] $^1$H-NMR (Resonanzsignal von $A^1$) [ppm] ($CDCl_3$, 250 MHz) |
|---|---|---|---|---|---|---|
| 1.014 | 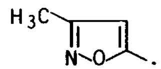 | $CH_2$ | H | $CH_2CH(CH_3)$ | 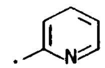 | 68-71 |
| 1.015 | 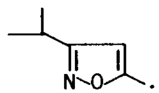 | $CH_2$ | H | $CH_2CH(CH_3)$ | 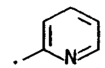 | δ = 5,02 |
| 1.016 | 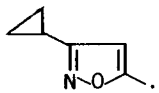 | $CH_2$ | H | $CH_2CH(CH_3)$ | 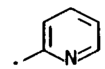 | 60-72 |
| 1.017 | 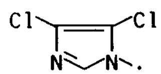 | $CH_2$ | H | $CH_2CH(CH_3)$ | 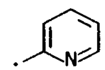 | δ = 5,57 |
| 1.018 | 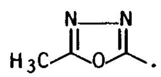 | $CH_2$ | H | $CH_2CH(CH_3)$ | 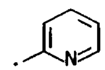 | δ = 5,33 |
| 1.019 | 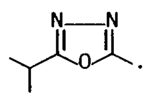 | $CH_2$ | H | $CH_2CH(CH_3)$ | 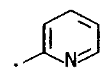 | δ = 5,15 |
| 1.020 | 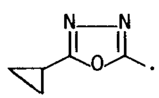 | $CH_2$ | H | $CH_2CH(CH_3)$ | 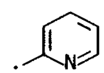 | δ = 5,12 |

Tabelle 1 (Fortsetzung)

| Nr. | X | $A^1$ | $R^1$ | $A^2$ | $R^2$ | physik. Daten Fp. [°C] $^1$H-NMR (Resonanzsignal von $A^1$) [ppm] ($CDCl_3$, 250 MHz) |
|---|---|---|---|---|---|---|
| 1.021 | 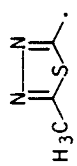 | $CH_2$ | H | $CH_2CH(CH_3)$ | 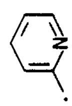 | 72-78 |
| 1.022 | 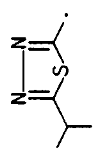 | $CH_2$ | H | $CH_2CH(CH_3)$ | 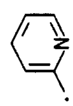 | $\delta$ = 5,39 |
| 1.023 | 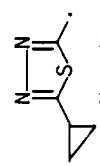 | $CH_2$ | H | $CH_2CH(CH_3)$ | 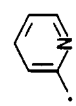 | $\delta$ = 5,33 |
| 1.024 | 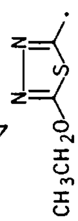 | $CH_2$ | H | $CH_2CH(CH_3)$ | 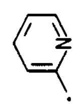 | 58-60 |

## Patentansprüche

**1.** Hydrochinondiether der allgemeinen Formel I,

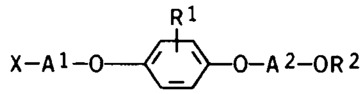

I

in der die Substituenten folgende Bedeutung haben:

$A^1$
Methylen, Ethylen oder Propylen, wobei diese Gruppen ein oder zwei $C_1$-$C_3$-Alkylreste tragen können;

$A^2$
Ethylen oder Propylen, wobei diese Gruppen ein oder zwei $C_1$-$C_3$-Alkylreste tragen können;

$R^1$
Wasserstoff, Halogen oder $C_1$-$C_6$-Alkyl;

$R^2$
1-Pyrazolyl, welches ein bis drei der folgenden Reste tragen kann: Halogen und $C_1$-$C_3$-Alkyl,

oder 2-, 3- oder 4-Pyridyl, welches ein bis drei der folgenden Reste tragen kann: Cyano, Nitro, Halogen, $C_1$-$C_3$-Alkyl, $C_1$-$C_2$-Halogenalkyl, $C_1$-$C_3$-Alkoxy, $C_1$-$C_2$-Halogenalkoxy, $C_1$-$C_3$-Alkylthio, $C_1$-$C_3$-Alkoxy-$C_1$-$C_3$-alkyl oder $C_3$-$C_6$-Cycloalkyl;

X
ein fünfgliedriger Heteroaromat, enthaltend ein bis drei Stickstoffatome und/oder ein Sauerstoff- oder Schwefelatom als Heteroatome, welcher ein bis drei der folgenden Reste tragen kann: Nitro, Halogen, $C_1$-$C_6$-Alkyl, $C_1$-$C_4$-Halogenalkyl, $C_1$-$C_6$-Alkoxy, $C_1$-$C_4$-Halogenalkoxy, $C_1$-$C_4$-Alkylthio, $C_1$-$C_4$-Halogenalkylthio, $C_1$-$C_3$-Alkoxy-$C_1$-$C_4$-alkyl, $C_3$-$C_8$-Cycloalkyl, $C_2$-$C_8$-Alkenyl, Aryl oder Aryl-$C_1$-$C_{10}$-Alkyl, wobei die aromatischen Reste ihrerseits ein bis fünf Halogenatome und/oder ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio.

**2.** Verfahren zur Herstellung der Verbindungen der Formel I gemäß Anspruch 1, dadurch gekennzeichnet, daß man einen 4-Hydroxyphenolether der allgemeinen Formel II,

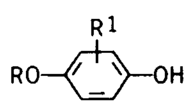

II

in der R eine inerte Schutzgruppe bedeutet, in an sich bekannter Weise in einem inerten organischen Lösungsmittel in Gegenwart einer Base mit einem Ethylen- oder Propylenderivat der allgemeinen Formel III,

$Y^1$-$A^2$-$Y^1$ III

in der $Y^1$ jeweils für eine nukleofuge Abgangsgruppe steht, zu einem Diether der allgemeinen Formel IVa

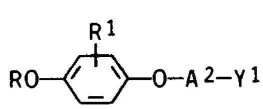

IVa

verethert, IVa anschließend in an sich bekannter Weise mit einer Hydroxyverbindung der allgemeinen Formel V

$HOR^2$ V

verethert, das so erhaltene Dietherderivat IVb

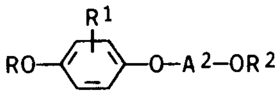

IVb

in an sich bekannter Weise durch Abspaltung der Schutzgruppe R in den entsprechenden Monoether der allgemeinen Formel VI

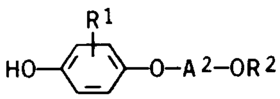

VI

überführt, welcher anschließend in an sich bekannter Weise mit einer Arylalkylverbindung der allgemeinen Formel VII

$X\text{-}A^1\text{-}Y^2$ VII

in der $Y^2$ für eine nukleofuge Abgangsgruppe steht, zu I verethert wird.

**3.** Diether der allgemeinen Formel IV

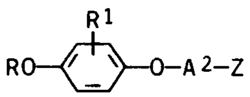

IV

in der $R^1$ und $A^2$ die in Anspruch 1 angegebene Bedeutung und die weiteren Substituenten folgende Bedeutung haben:

R
$C_1$-$C_6$-Alkyl;

Benzyl, wobei der aromatische Rest ein bis fünf Halogenatome und/oder ein bis drei der folgenden Gruppen tragen kann: Cyano, Nitro, $C_1$-$C_4$-Alkyl, $C_1$-$C_4$-Alkoxy oder $C_1$-$C_4$-Alkylthio;

Silyl, welches drei der folgenden Reste trägt: $C_1$-$C_6$-Alkyl und/oder Phenyl, wobei der aromatische Rest ein bis fünf Halogenatome und/oder ein bis drei der folgenden Gruppen tragen kann: Cyano, Nitro, $C_1$-$C_4$-Alkyl, $C_1$-$C_4$-Alkoxy oder $C_1$-$C_4$-Alkylthio;

Z
Hydroxy; Halogen;

$C_1$-$C_{10}$-Alkylsulfonyl, Phenylsulfonyl, wobei der aromatische Rest ein bis fünf Halogenatome und/oder ein bis drei der folgenden Gruppen tragen kann: Cyano, Nitro, $C_1$-$C_4$-Alkyl, $C_1$-$C_4$-Alkoxy oder $C_1$-$C_4$-Alkylthio;

eine Pyrazolyl-1-oxy-gruppe, welche ein bis drei der folgenden Reste tragen kann: Halogen und $C_1$-$C_3$-Alkyl,

eine Pyridyl-2-, -3- oder -4-oxygruppe, welche ein bis drei der folgenden Reste tragen kann: Cyano, Nitro, Halogen, $C_1$-$C_3$-Alkyl, $C_1$-$C_2$-Halogenalkyl, $C_1$-$C_3$-Alkoxy, $C_1$-$C_2$-Halogenalkoxy, $C_1$-$C_3$-Alkylthio, $C_1$-$C_3$-Alkoxy-$C_1$-$C_3$-alkyl oder $C_3$-$C_6$-Cycloalkyl.

4. Verfahren zur Herstellung der Verbindungen der Formel I gemäß Anspruch 1, in denen $R^2$ für 2-, 3- oder 4-Pyridyl steht, dadurch gekennzeichnet, daß man einen Diether der allgemeinen Formel IV gemäß Anspruch 3, in der Z für Hydroxy steht, in an sich bekannter Weise in einem inerten organischen Lösungsmittel in Gegenwart einer Base mit einem Pyridinderivat der allgemeinen Formel IX

   Hal-$R^2$  IX

   in der Hal für ein Halogenatom und $R^2$ für ein Pyridyl steht, verethert.

5. Verfahren zur Herstellung der Diether der allgemeinen Formel IV gemäß Anspruch 3, in der Z Hydroxy bedeutet, dadurch gekennzeichnet, daß man einen 4-Hydroxyphenolether II gemäß Anspruch 2 in an sich bekannter Weise in einem inerten organischen Lösungsmittel in Gegenwart einer Base mit einem cyclischen Carbonat der allgemeinen Formel VIII

   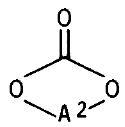 VIII

   umsetzt.

6. Monoether der allgemeinen Formel VI

   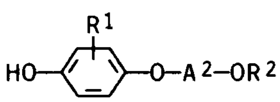 VI

   in der die Substituenten $R^1$, $R^2$ und $A^2$ die in Anspruch 1 gegebene Bedeutung haben.

7. Schädlingsbekämpfungsmittel, enthaltend eine wirksame Menge einer Verbindung der allgemeinen Formel I gemäß Anspruch 1 und inerte Zusatzstoffe.

8. Verfahren zur Bekämpfung von Schädlingen, dadurch gekennzeichnet, daß man die Schädlinge und/oder ihren Lebensraum mit einer wirksamen Menge einer Verbindung der allgemeinen Formel I gemäß Anspruch 1 behandelt.

**Patentansprüche für folgende Vertragsstaat: ES**

1. Schädlingsbekämpfungsmittel, enthaltend eine wirksame Menge eines Hydrochinondiether der allgemeinen Formel I,

   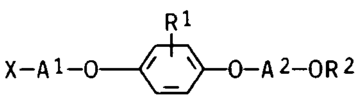 I

   in der die Substituenten folgende Bedeutung haben:

   $A^1$
   Methylen, Ethylen oder Propylen, wobei diese Gruppen ein oder zwei $C_1$-$C_3$-Alkylreste tragen können;

   $A^2$
   Ethylen oder Propylen, wobei diese Gruppen ein oder zwei $C_1$-$C_3$-Alkylreste tragen können;

   $R^1$

Wasserstoff, Halogen oder $C_1$-$C_6$-Alkyl;

$R^2$
1-Pyrazolyl, welches ein bis drei der folgenden Reste tragen kann: Halogen und $C_1$-$C_3$-Alkyl,

oder 2-, 3- oder 4-Pyridyl, welches ein bis drei der folgenden Reste tragen kann: Cyano, Nitro, Halogen, $C_1$-$C_3$-Alkyl, $C_1$-$C_2$-Halogenalkyl, $C_1$-$C_3$-Alkoxy, $C_1$-$C_2$-Halogenalkoxy, $C_1$-$C_3$-Alkylthio, $C_1$-$C_3$-Alkoxy-$C_1$-$C_3$-alkyl oder $C_3$-$C_6$-Cycloalkyl;

X
ein fünfgliedriger Heteroaromat, enthaltend ein bis drei Stickstoffatome und/oder ein Sauerstoff- oder Schwefelatom als Heteroatome, welcher ein bis drei der folgenden Reste tragen kann: Nitro, Halogen, $C_1$-$C_6$-Alkyl, $C_1$-$C_4$-Halogenalkyl, $C_1$-$C_6$-Alkoxy, $C_1$-$C_4$-Halogenalkoxy, $C_1$-$C_4$-Alkylthio, $C_1$-$C_4$-Halogenalkylthio, $C_1$-$C_3$-Alkoxy-$C_1$-$C_4$-alkyl, $C_3$-$C_8$-Cycloalkyl, $C_2$-$C_8$-Alkenyl, Aryl oder Aryl-$C_1$-$C_{10}$-Alkyl, wobei die aromatischen Reste ihrerseits ein bis fünf Halogenatome und/oder ein bis drei der folgenden Gruppen tragen können: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio,

und inerte Zusatzstoffe.

**2.** Verfahren zur Herstellung der Verbindungen der Formel I gemäß Anspruch 1, dadurch gekennzeichnet, daß man einen 4-Hydroxyphenolether der allgemeinen Formel II,

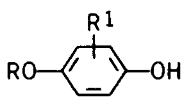

II

in der R eine inerte Schutzgruppe bedeutet, in an sich bekannter Weise in einem inerten organischen Lösungsmittel in Gegenwart einer Base mit einem Ethylen- oder Propylenderivat der allgemeinen Formel III,

$Y^1$-$A^2$-$Y^1$ III

in der $Y^1$ jeweils für eine nukleofuge Abgangsgruppe steht, zu einem Diether der allgemeinen Formel IVa

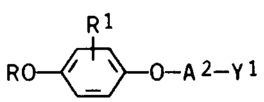

IVa

verethert, IVa anschließend in an sich bekannter Weise mit einer Hydroxyverbindung der allgemeinen Formel V

$HOR^2$ V

verethert, das so erhaltene Dietherderivat IVb

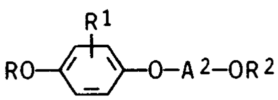

IVb

in an sich bekannter Weise durch Abspaltung der Schutzgruppe R in den entsprechenden Monoether der allgemeinen Formel VI

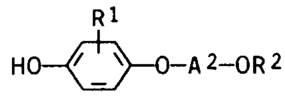

VI

überführt, welcher anschließend in an sich bekannter Weise mit einer Arylalkylverbindung der allgemeinen Formel VII

$X\text{-}A^1\text{-}Y^2$ VII

in der $Y^2$ für eine nukleofuge Abgangsgruppe steht, zu I verethert wird.

**3.** Verfahren zur Herstellung der Verbindungen der Formel I gemäß Anspruch 1, in denen $R^2$ für 2-, 3- oder 4-Pyridyl steht, dadurch gekennzeichnet, daß man einen Diether der allgemeinen Formel IV

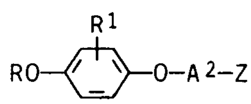

IV

in der $R^1$ und $A^2$ die in Anspruch 1 angegebene Bedeutung und die weiteren Substituenten folgende Bedeutung haben:

R
$C_1$-$C_6$-Alkyl;

Benzyl, wobei der aromatische Rest ein bis fünf Halogenatome und/oder ein bis drei der folgenden Gruppen tragen kann: Cyano, Nitro, $C_1$-$C_4$-Alkyl, $C_1$-$C_4$-Alkoxy oder $C_1$-$C_4$-Alkylthio;

Silyl, welches drei der folgenden Reste trägt: $C_1$-$C_6$-Alkyl und/oder Phenyl, wobei der aromatische Rest ein bis fünf Halogenatome und/oder ein bis drei der folgenden Gruppen tragen kann: Cyano, Nitro, $C_1$-$C_4$-Alkyl, $C_1$-$C_4$-Alkoxy oder $C_1$-$C_4$-Alkylthio;

Z
Hydroxy;

in an sich bekannter Weise in einem inerten organischen Lösungsmittel in Gegenwart einer Base mit einem Pyridinderivat der allgemeinen Formel IX

$Hal\text{-}R^2$ IX

in der Hal für ein Halogenatom und $R^2$ für ein Pyridyl steht, verethert.

**4.** Verfahren zur Herstellung der Diether der allgemeinen Formel IV gemäß Anspruch 3, dadurch gekennzeichnet, daß man einen 4-Hydroxyphenolether II gemäß Anspruch 2 in an sich bekannter Weise in einem inerten organischen Lösungsmittel in Gegenwart einer Base mit einem cyclischen Carbonat der allgemeinen Formel VIII

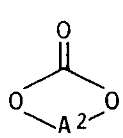

VIII

umsetzt.

5. Verfahren zur Bekämpfung von Schädlingen, dadurch gekennzeichnet, daß man die Schädlinge und/oder ihren Lebensraum mit einer wirksamen Menge einer Verbindung der allgemeinen Formel I gemäß Anspruch 1 behandelt.